# EUROPEAN PATENT APPLICATION

(11) **EP 4 545 701 A1**
(43) Date of publication of application: **30.04.2025**
(21) Application number: 23831790.3
(22) Date of filing: 20.06.2023
(51) Int. Cl.: D06F 58/20, D06F 58/24, D06F 58/26, D06F 58/10, D06F 73/02, D06F 39/00, A61L 2/07

(54) **CLOTHING TREATMENT APPARATUS**

(30) Priority: 30.06.2022 KR 20220080292
(71) Applicant: LG Electronics Inc., Yeongdeungpo-gu Seoul 07336 (KR)
(72) Inventor: KANG, Hyungha, Seoul 08592 (KR); KIM, Jaehyung, Seoul 08592 (KR)
(74) Representative: Vossius & Partner Patentanwälte Rechtsanwälte mbB
(86) International application number: PCT/KR2023/008508
(87) International publication number: WO 2024/005430

(57) **Abstract**

The present disclosure relates to a clothing treatment apparatus in which a residual water treatment unit, by means of which water condensed in a circulation duct accommodating a heat exchanger assembly is collected in a drain tank, communicates the drain tank and the inside of the circulation duct so that some of the water collected in the drain tank can be discharged back into the circulation duct, thus making it possible to prevent the water from leaking out even when the drain tank is full.

## Description

### [Technical Field]

The present disclosure relates to a laundry treating apparatus. More specifically, the present disclosure relates to a laundry treating apparatus that may collect water condensed from air and steam that have passed through laundry in a detachable drainage tank.

### [Background]

In general, a laundry treating apparatus is a concept that includes a washing machine that soaks laundry in water so as to be in a wet state and then removes foreign substances via a chemical action of detergent and a physical action such as drum rotation, and a dryer that dries the wet laundry using hot air and steam.

However, recently, a laundry manager that keeps the laundry in a comfortable and clean state without soaking the laundry in a dry state in water has appeared. Such laundry manager is able to perform a refreshing cycle by supplying steam or hot air while the laundry is mounted to deodorize the laundry and dry or sterilize the laundry.

Such laundry manager is able to optionally add fragrance to the laundry, and has recently taken up a significant proportion in the laundry treating apparatus along with the washing machine and the dryer.

Because the laundry manager that performs the refreshing cycle of the laundry accommodates the dry laundry therein, a steam supply that supplies steam to the laundry is essential.

Examples of such existing laundry treating apparatus include Korean Patent Application Publication Nos. 10-2020-0057545 and 10-2011-0067832.

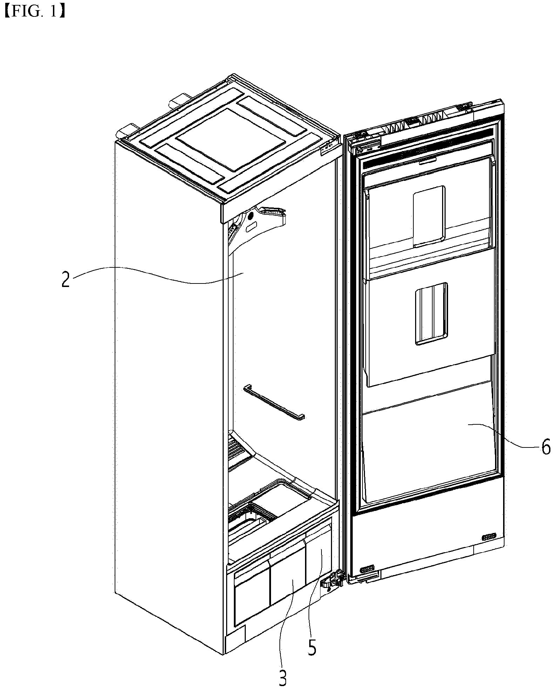

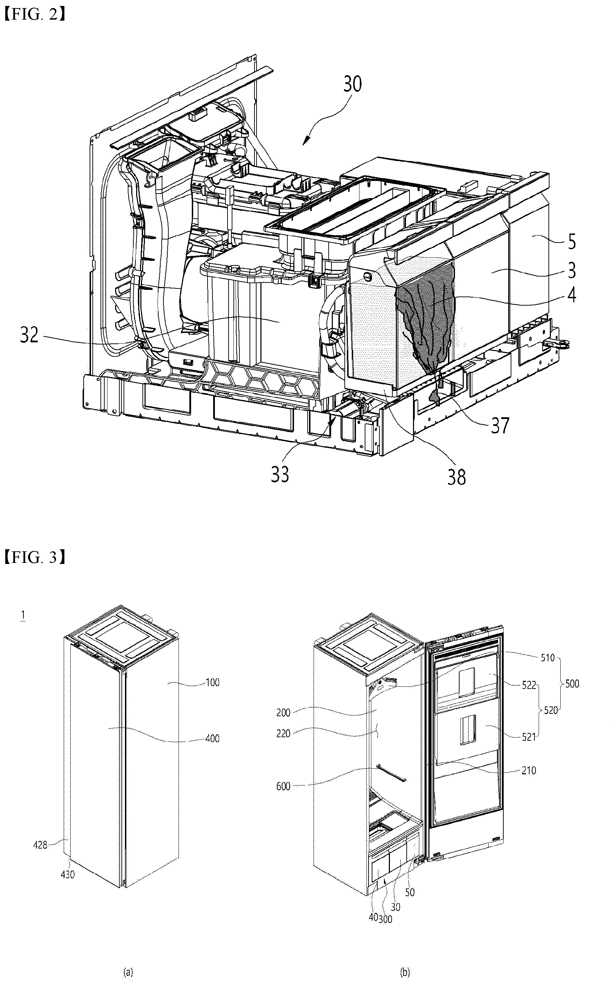

FIG. 1 shows a basic structure of an existing laundry treating apparatus, and FIG. 2 shows a machine room and a residual water treatment structure of an existing laundry treating apparatus.

Referring to FIG. 1, the existing laundry treating apparatus may include a cabinet 2 having an accommodating space where the laundry is mounted, a door 6 that opens and closes the cabinet 2, and a machine room 30 disposed at a lower portion of the cabinet 2.

The machine room 30 may include a water supply tank 3 that stores therein water for generating steam inside the cabinet 2, and a drainage tank 4 that collects moisture condensed from the steam or the laundry, and may additionally include a drawer 5 providing a space for storing an additional necessary item or the like therein.

Referring to FIG. 2, the machine room 30 may further include a circulation duct 32 that circulates air inside the cabinet 2, and a seating stand 38 that is disposed in front of the circulation duct 32 and is able to seat the water supply tank 3, the drainage tank 4, and the drawer 5 thereon.

The circulation duct 32 may be installed with an evaporator that cools air introduced from inside the cabinet 2 to condense moisture, and a heat exchanger such as a condenser that heats the cooled air.

Water condensed in the circulation duct 32 may be collected along a bottom surface of the circulation duct 32 and collected in the drainage tank 4 via a pump 33. Thus, the existing laundry treating apparatus may continuously perform drying/sterilization/refreshing of the laundry accommodated inside the cabinet 2 via steam and hot air by removing water from air that has treated the laundry and resupplying the same.

In the existing laundry treating apparatus, the drainage tank 4 is generally not in communication with an external drain hole or the like, but is equipped as a container that simply stores water. This increases convenience of installation because the existing laundry treating apparatus does not need to be adjacent to the drain hole or the like.

However, the existing laundry treating apparatus has a limitation that water collected in the drainage tank 4 is not able to be automatically discharged unless a user directly discharges the same.

When the drainage tank 4 is full, it is difficult to collect water in the drainage tank 4, and thus, water collected in the drainage tank 4 leaks out of the machine room 30.

Furthermore, even when the drainage tank 4 is not full at the beginning, when the drainage tank 4 becomes full during the laundry treatment process, water introduced into the drainage tank 4 leaks out.

In addition, when the laundry treating apparatus is set to stop operating when the drainage tank 4 is full to prevent the water leakage from the drainage tank 4, the user is not able to treat the laundry at a desired time point.

In particular, when the operation of the laundry treating apparatus is stopped when the drainage tank 4 is full, the operation of the laundry treating apparatus is stopped even though the laundry treatment is not completed, thereby damaging a reliability of the product.

In one example, even when placement of a backflow allowing portion that guides water flowing back from the drainage tank 4 back to the circulation duct 32 is considered, the existing laundry treating apparatus is not able to provide a method to solve such problem other than placing the backflow allowing portion as a component separate from the circulation duct or placing the backflow allowing portion outside the circulation duct.

### [Summary]

### [Technical Problem]

The present disclosure is to provide a laundry treating apparatus including a backflow allowing portion that guides water that flows back or is discharged from a drainage tank into a circulation duct where air circulates, wherein a flow channel forming the backflow allowing portion is formed integrally with the circulation duct.

The present disclosure is to provide a laundry treating apparatus in which the backflow allowing portion may be manufactured or formed integrally with the circulation duct.

The present disclosure is to provide a laundry treating apparatus in which the backflow allowing portion may be fixed to the circulation duct.

The present disclosure is to provide a laundry treating apparatus that may block water from leaking to the circulation duct or out of a machine room even when the drainage tank is full.

The present disclosure is to provide a laundry treating apparatus that may block water from overflowing from the drainage tank even when the drainage tank is full during laundry treatment.

The present disclosure is to provide a laundry treating apparatus that may still supply steam to the laundry and manage the laundry even when the drainage tank is full.

The present disclosure is to provide a laundry treating apparatus that may continuously supply water to the drainage tank even when the drainage tank is full.

The present disclosure is to provide a laundry treating apparatus that may block water from overflowing outside the machine room even when water overflows from the drainage tank.

The present disclosure is to provide a laundry treating apparatus that may circulate water in the drainage tank to the circulation duct where air circulates.

### [Technical Solutions]

In the present disclosure, a residual water treater that collects water condensed from a circulation duct where a heat exchanger is accommodated into a drainage tank may allow the drainage tank and inside of the circulation duct to be in communication with each other. The drainage tank may be disposed outside the circulation duct.

As a result, a portion of water collected in the drainage tank may be dischargeable back into the circulation duct.

Specifically, the residual water treater may include a backflow allowing portion extending through one surface of the circulation duct and in communication with the drainage tank.

The backflow allowing portion may be disposed at a certain height apart from a bottom surface of the drainage tank, and may be disposed closer to a top surface of the drainage tank.

The residual water treater may include a drainage pump that allows water collected on a bottom surface of the circulation duct to flow, and a discharge pipe in communication with the drainage pump and the drainage tank to inject water into the drainage tank, and the backflow allowing portion may be equipped separately from the discharge pipe.

That is, the backflow allowing portion and the discharge pipe may be blocked from being in direct communication with each other. Water introduced into the discharge pipe may first be introduced into the drainage tank without directly flowing back into the backflow allowing portion.

The residual water treater may further include a recovery flow channel extending from the circulation duct to be inserted into the drainage tank to allow the backflow allowing portion and the drainage tank to be in communication with each other.

The backflow allowing portion may include a flow channel assembly extending in a width direction of an inner surface of the circulation duct to guide water to a bottom surface of the circulation duct.

The circulation duct may include a reservoir recessed from the bottom surface thereof to collect water therein, the recovery flow channel may be disposed close to one side of the inner surface of the circulation duct, the reservoir may be disposed close to an opposite side to the one side of the inner surface of the circulation duct, and the flow channel assembly may extend from the backflow allowing portion toward the reservoir.

The flow channel assembly may include a guide flow channel extending to be lowered along the width direction of the inner surface of the circulation duct.

The flow channel assembly may include an inflow flow channel extending from the communication hole to one end of the guide flow channel, and a discharge flow channel extending from the other end of the guide flow channel to the bottom surface of the circulation duct.

The discharge flow channel may be set to have a steeper inclination than the guide flow channel, and the flow channel assembly may further include a flow channel restricting portion protruding from a bottom surface of the guide flow channel and inducing water flowing along the guide flow channel to flow toward the discharge flow channel.

To solve the above-described problems, the present disclosure may place the residual water treater on a front surface of the circulation duct facing the heat exchanger.

The residual water treater may include a backflow allowing portion extending through the front surface of the circulation duct and in communication with the drainage tank, and a blocking portion blocking water introduced into the backflow allowing portion from flowing to the evaporator.

The residual water treater may further include a flow channel assembly disposed on the front surface of the circulation duct to guide water introduced into the backflow allowing portion to a bottom surface of the circulation duct.

The blocking portion may be disposed along an inner surface of the flow channel assembly to block water flowing along the flow channel assembly from being introduced into the evaporator.

The blocking portion may include a recovery partition wall extending from one surface of the flow channel assembly to face the backflow allowing portion, and a flow rate adjusting portion extending from the recovery partition wall toward the front surface of the circulation duct to collect a portion of water introduced from the communication hole.

### [Advantageous Effects]

The present disclosure minimizes a cost of manufacturing the circulation duct and the backflow allowing portion.

The present disclosure omits a process of manufacturing or installing the backflow allowing portion separately from the circulation duct.

The present disclosure minimizes a space in which the circulation duct and the backflow allowing portion are disposed.

The present disclosure blocks a possibility of the backflow allowing portion being separated from the circulation duct.

The present disclosure blocks a possibility of water leaking from the backflow allowing portion.

The present disclosure blocks water from leaking out of the machine room where the drainage tank is installed even when the drainage tank that collects residual water is full.

The present disclosure blocks water from overflowing from the drainage tank even when the drainage tank is full during the laundry treatment.

The present disclosure still supplies steam to the laundry and manages the laundry even when the drainage tank is full.

The present disclosure continuously supplies water to the drainage tank even when the drainage tank is full.

The present disclosure blocks water from overflowing outside the machine room even when water overflows from the drainage tank.

The present disclosure circulates water in the drainage tank to the circulation duct where air circulates.

### [Brief Description of the Drawings]

FIG. 1 shows an existing laundry treating apparatus.
FIG. 2 shows a drainage structure of an existing laundry treating apparatus.
FIG. 3 shows an outer appearance of a laundry treating apparatus of the present disclosure.
FIG. 4 shows a machine room structure of a laundry treating apparatus of the present disclosure.
FIG. 5 shows a machine room base structure of a laundry treating apparatus of the present disclosure.
FIG. 6 shows a circulation duct structure of a laundry treating apparatus of the present disclosure.
FIG. 7 illustrates a shape of a circulation duct of a laundry treating apparatus of the present disclosure.
FIG. 8 is a cross-sectional view of the circulation duct.
FIG. 9 shows a structure of a reservoir of a laundry treating apparatus of the present disclosure in detail.
FIG. 10 is a cross-sectional view (S-S') of a circulation duct cut in a height direction.
FIG. 11 shows an inclined structure related to a reservoir.
FIG. 12 shows structures of the reservoir and a residual water treater.
FIG. 13 shows an embodiment of a water cover.
FIG. 14 shows a state in which a water cover is installed in a circulation duct.
FIG. 15 shows a detailed structure of a water cover.
FIG. 16 shows a structure of a controller installation portion disposed on a base of a laundry treating apparatus of the present disclosure.
FIG. 17 shows a structure of an air discharger 323 of a laundry treating apparatus of the present disclosure.
FIG. 18 shows a structure of a base cover of a laundry treating apparatus of the present disclosure.
FIG. 19 shows a structure of an outside air duct.
FIG. 20 shows a flow of air flowing through a circulation duct.
FIG. 21 shows an installation structure of a steam supply.
FIG. 22 shows a detailed structure of a steam supply.
FIG. 23 shows inside of a steam casing.
FIG. 24 shows an embodiment of a residual water treater of a laundry treating apparatus of the present disclosure.
FIG. 25 shows a water supply and storage structure of a laundry treating apparatus of the present disclosure.
FIG. 26 shows a residual water collection structure of a laundry treating apparatus of the present disclosure.
FIG. 27 shows a leak prevention structure of a laundry treating apparatus of the present disclosure.
FIG. 28 shows a structure in which water is introduced into a circulation duct when water overflows from a drainage tank of a laundry treating apparatus of the present disclosure.
FIG. 29 shows a structure in which water introduced into a circulation duct is guided.

### [Best Mode]

Hereinafter, embodiments disclosed herein will be described in detail with reference to the attached drawings. In the present document, identical or similar components are assigned identical or similar reference numerals even in different embodiments, and descriptions thereof are replaced with the first description. A singular expression used herein includes a plural expression unless the context clearly indicates otherwise. In addition, when describing the embodiments disclosed herein, when it is determined that a detailed description of a related known technology may obscure the gist of the embodiments disclosed herein, the detailed description thereof will be omitted. In addition, it should be noted that the attached drawings are only intended to facilitate easy understanding of the embodiments disclosed herein, and the technical ideas disclosed herein should not be construed as being limited by the attached drawings.

FIG. 3 shows an outer appearance of a laundry treating apparatus 1 of the present disclosure.

Referring to (a) in FIG. 3, the laundry treating apparatus of the present disclosure may include a cabinet 100 forming the outer appearance thereof, and a door 400 pivotably coupled to the cabinet 100.

The door 400 may include a main body 410 forming a front surface of the cabinet 100, and an installation body 420 extending from one side of the main body 410 and on which a display for displaying information of the laundry treating apparatus may be installed.

The installation body 420 may form a step 430 from the main body 410 in a rearward direction of the cabinet 100.

In one example, at least a portion of the installation body 420 may be disposed at the rear of the main body 410 so as to overlap the main body 410 in a front and rear direction. Accordingly, the step 430 may serve as a handle.

The installation body 420 may be made of a material or may have a color different from that of the main body 410. In addition, the installation body 420 may be made of a translucent material through which light emitted from the display may be transmitted.

Referring to (b) in FIG. 3, an inner casing 200 having an accommodating space 220 for accommodating laundry may be disposed inside the cabinet 100. The inner casing 200 may have an opening 210 at a front side thereof through which the laundry enters and exits, and the opening 210 may be shielded by the door 400.

The inner casing 200 may be made of a plastic resin-based material, and may be made of a reinforced plastic resin-based material that does not deform even with air at a temperature higher than a room temperature or heated air (hereinafter, hot air) and steam or moisture.

The inner casing 200 may have a height greater than a width. Accordingly, the laundry may be accommodated in the accommodating space 220 without being folded or wrinkled.

The laundry treating apparatus 1 of the present disclosure may include a mounter 500 that may mount the laundry in the accommodating space 220 of the inner casing 200.

The mounter 500 may include a hanger 510 disposed on a top surface of the inner casing 200 to mount the laundry thereon.

When the laundry is mounted on the hanger 510, the laundry may be placed in a state of floating in air inside the accommodating space 220.

In one example, the above mounter 500 may further include a pressurizer 520 that may be coupled to an inner surface of the door 400 to fix the laundry.

The hanger 510 may be formed in a bar shape disposed along a width direction of the inner casing 200 to support a clothes hanger on which the laundry is mounted. In addition, as illustrated, the hanger 510 may be formed in the clothes hanger shape to enable the laundry to be directly mounted thereon.

The laundry treating apparatus of the present disclosure may further include an oscillator that vibrates the hanger 510 to remove foreign substances such as fine dust attached to the laundry.

The mounter 500 may include the pressurizer 520 that may be disposed on the door 400 and may pressurize and fix the laundry. The pressurizer 520 may include a support 522 that is fixed to the inner surface of the door 400 and supports one surface of the laundry, and a press 521 that pressurizes the laundry supported on the support 522.

The press 521 may move toward or away from the support 522. For example, the press 521 may be pivotably disposed on the support 522 or the inner surface of the door 400.

Therefore, the press 521 and the support 522 may pressurize both surfaces of the laundry to remove wrinkles from the laundry and create intended creases.

The laundry treating apparatus of the present disclosure may be equipped with a machine room 300 in which various apparatuses that may supply at least one of hot air and steam to the accommodating space 220 or purify or dehumidify outside air of the cabinet 100 are installed.

The machine room 300 may be disposed separately or partitioned from the inner casing 200, but may be in communication with the inner casing 200.

The machine room 300 may be disposed under the inner casing 200. Accordingly, when hot air and steam having low specific gravities are supplied to the inner casing 200, hot air and steam may be naturally supplied to the laundry.

The machine room 300 may include a heat supply 340 that may supply hot air into the inner casing 200. The heat supply 340 may be equipped as a heat pump system, or may be equipped as a heater that directly heats air with electric energy.

When the heat supply 340 is equipped as the heat pump system, it may dehumidify and heat air discharged from the inner casing 200 again and supply air to the inner casing 200. A detailed structure thereof will be described later.

The machine room 300 may include a steam supply 800 that may supply steam into the inner casing 200. The steam supply 800 may be equipped to directly supply steam into the inner casing 200. A detailed structure thereof will be described later.

To this end, the inner casing 200 may include a plurality of through-holes 230 that extend through one surface thereof and are in communication with the machine room 300.

Via the through-holes 230, air in the accommodating space 220 may be supplied to the machine room 300, and at least one of hot air and steam generated in the machine room 300 may be supplied to the accommodating space 200.

The through-holes 230 may include an inflow hole 231 extending through a bottom surface of the inner casing 200 and through which air inside the inner casing 200 is discharged or sucked into the machine room 300, and an exhaust hole 232 extending through the bottom surface of the inner casing 200 and through which hot air generated in the machine room 300 is discharged.

The exhaust hole 232 may be defined in the bottom surface of the inner casing 200 so as to be biased toward a rear surface of the inner casing 200. For example, the exhaust hole 232 may be defined so as to be inclined with respect to the ground between the bottom surface and the rear surface of the inner casing 200 and to face the hanger 510.

In addition, the inflow hole 231 may be defined in the bottom surface of the inner casing 200 so as to be biased forward. Accordingly, the inflow hole 231 may be defined to be spaced apart from the exhaust hole 232.

The through-holes 230 may include a steam hole 233 to which steam generated from the steam supply 800 is supplied. The steam hole 233 may be defined on one side of the exhaust hole 232.

In one example, a water supply tank 30 that may supply water to the steam supply 800 and a drainage tank 40 in which condensate condensed in the heat supply 340 is collected may be disposed at a front side of the machine room 300.

The water supply tank 30 and the drainage tank 40 may be detachably disposed at the front side of the machine room 300. Accordingly, the laundry treating apparatus 1 of the present disclosure may be freely installed without being restricted by a water supply source or a drainage source.

In one example, a drawer 50 that is extended forward and has a separate accommodating space may be further disposed at the front side of the machine room 300. The drawer 50 may store a steam generator or an iron therein.

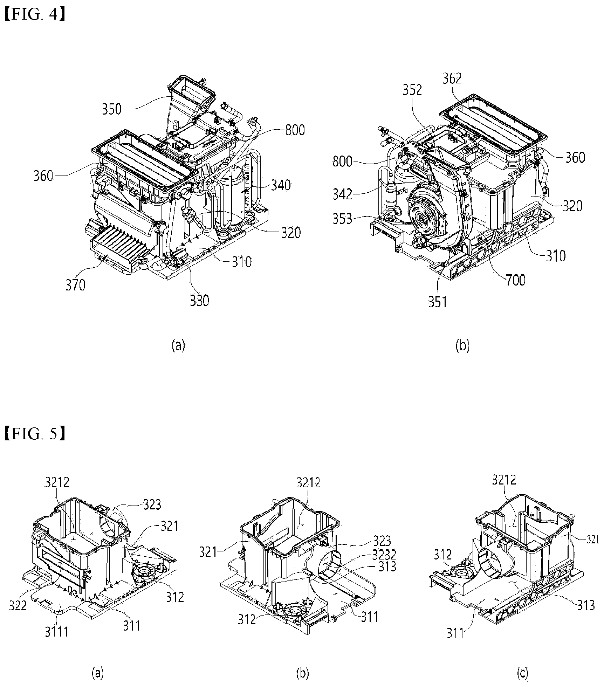

FIG. 4 shows a machine room structure of a laundry treating apparatus of the present disclosure.

(a) in FIG. 4 is a front view of the machine room 300, and (b) in FIG. 4 is a rear view of the machine room 300.

Inside the machine room 300, components for supplying hot air to a laundry treatment space, circulating air inside the laundry treatment space, supplying steam to the laundry treatment space, or purifying air outside the cabinet may be disposed.

The machine room 300 may include a base 310 that defines a space in which various apparatuses are supported or installed. The base 310 may provide an area size for the various apparatuses to be installed.

The base 310 may be installed with a circulation duct 320 through which air introduced from the inner casing 200 or the outside of the cabinet 100 flows.

The circulation duct 320 may be formed in a casing shape with an open top surface, and some components of the heat supply 340 may be installed inside the circulation duct 320.

When the heat supply 340 is equipped as the heat pump system, the circulation duct 320 may include heat exchangers 341 and 342 to be described below and a compressor 342 that supplies a high-temperature and high-pressure refrigerant to the heat exchanger.

The heat exchangers 341 and 342 may be accommodated inside the circulation duct 320 to cool and dehumidify air flowing through the circulation duct 320, or may heat the air to generate hot air.

When the circulation duct 320 is equipped to suck air outside the cabinet 100, an outside air duct 370 that sucks outside air may be installed in front of the circulation duct 320.

The circulation duct 320 may be in communication with the outside air duct 370 and may selectively suck outside air.

The water supply tank and the drainage tank may be detachably coupled to a front surface of the circulation duct 320. The water supply tank 30 and the drainage tank 40 may be seated and disposed on the outside air duct 370.

The circulation duct 320 may be coupled to the base 310, but may be formed integrally with the base 310. For example, the base 310 and the circulation duct 320 may be manufactured via injection-molding.

The machine room 300 may include a base cover 360 that allows the circulation duct 320 and the inflow hole 231 to be in communication with each other.

The base cover 360 may be coupled to an upper portion of the circulation duct 320 to guide air sucked from the inflow hole 231 into the circulation duct 320.

The base cover 360 may block air inside the circulation duct 320 from being discharged to the outside by shielding a top surface of the circulation duct 320. A lower portion of the base cover 360 and the top surface of the circulation duct 320 may form one surface of a flow channel of the circulation duct 320.

The base cover 360 may include an inlet 362 connecting the inflow hole 231 with the circulation duct 320. The inlet 362 may be formed in a duct shape to serve as an intake duct that delivers air inside the inner casing 200 to the circulation duct 320.

The steam supply 800 that is connected to the water supply tank 30 to receive water, generate steam, and supply steam to the inner casing 200 may be installed in the machine room 300. The steam supply 800 may be seated and disposed on the base cover 360.

The steam supply 800 may be disposed at the rear of the inlet 362.

The machine room 300 may include a fan installation portion 350 that is in communication with the circulation duct 320 and the inner casing 200. The fan installation portion 350 may include a blower fan 353 that provides power for air inside the circulation duct 320 to flow in one direction, and a fan housing 351 that accommodates the blower fan 353 therein and is coupled to or extended from the circulation duct 320.

The fan installation portion 350 may include an exhaust duct 352 that allows the circulation duct 320 and the exhaust hole 232 to be in communication with each other.

The exhaust duct 352 may extend from the fan housing 351 toward the exhaust hole 232 with an area size of a cross-section corresponding to the exhaust hole 232.

As a result, air inside the inner casing 200 may be introduced via the base cover 360, then pass through the circulation duct 320, and then be supplied back into the inner casing 200 via the fan installation portion 350.

In one example, the base 310 may include a compressor installation portion 313 in which the compressor 342 that supplies the refrigerant to the heat exchangers 341 and 343 is installed. The compressor installation portion 313 may be disposed outside the circulation duct 320.

In addition, a controller or a control panel 700 that controls the laundry treating apparatus of the present disclosure may be installed on the base 310.

The base 310 may include a controller installation portion 312 that defines a space in which the controller 700 may be inserted under the circulation duct 320.

The controller 700 may control all electronically controlled components such as the compressor 342, the steam supply 800, and the blower fan 353.

Because the controller 700 is inserted and supported in the base 310, vibration or impact applied to the controller 700 may be buffered. In addition, because the controller 700 is disposed close to all of the electronic components, occurrence of control errors such as noise may be minimized.

In addition, the steam supply is disposed on the circulation duct 320, and the controller 700 is disposed under the circulation duct 320. Therefore, the circulation duct 320 may be formed in a straight duct shape between the steam supply 800 and the controller 700. Therefore, a flow resistance of air passing through the circulation duct 320 may be minimized.

The circulation duct 320, the outside air duct 370, the steam supply 800, the controller 700, and the heat supply 340 may be equipped in a module format on the base 310.

Accordingly, the base 310 may be easily installed and maintained while being extended forward from and retracted rearward into the machine room 300.

FIG. 5 shows a machine room base structure of a laundry treating apparatus of the present disclosure.

(a) in FIG. 5 is a front perspective view of the base 310, and (b) and (c) in FIG. 5 are rear perspective views of the base 310.

The base 310 may be installed on a base plate forming a bottom surface of the laundry treating apparatus. The base 310 may itself form the bottom surface of the laundry treating apparatus.

The base 310 may include a base bottom 311 forming a support surface. The base bottom 311 may form the bottom surface of the laundry treating apparatus. In addition, the base bottom 311 may be installed on a top surface of a bottom surface of the cabinet 100 forming the bottom surface of the laundry treating apparatus.

The base 310 may be integrally formed with the circulation duct 320 forming at least a portion of the flow channel through which air flows. The circulation duct 320 may be formed by extending upward from the base bottom 311.

The circulation duct 320 may include a duct body 321 that extends from the base bottom 311 to form the flow channel, a heat exchanger installation portion 3212 that provides a space in which an evaporator 341 or a condenser 343 is installed inside the duct body 321, and an air discharger 323 that is disposed at the rear of the duct body 321 and through which air of the duct body 321 is discharged.

The air discharger 323 may be formed in a pipe shape that extends rearward from the duct body 321. A diameter of the air discharger 323 may be smaller than a width of the duct body 321.

The air discharger 323 may be connected to a fan housing 350. Air discharged from the air discharger 323 may be guided into the inner casing 200 via the fan housing 350.

The circulation duct 320 may include an outside air intake portion 322 formed by extending through a front surface of the duct body 321.

The outside air intake portion 322 may be in communication with the outside air duct 370. The outside air duct 370 may be supported by being seated in front of the outside air intake portion 322.

The circulation duct 320 may include a damper that opens and closes the outside air intake portion 322. Introduction of outside air into the circulation duct 320 may be allowed or blocked via the opening and closing of the damper.

The base 310 may include a compressor installation portion 312 that provides a space in which the compressor 342 is installed. The compressor installation portion 312 may be formed at one side of the base bottom 311 and may be formed integrally with the base bottom 311.

The compressor installation portion 312 may be formed with a protrusion that may support the compressor 342. The compressor installation portion 312 may be disposed to be biased rearward on the base 310. The compressor installation portion 312 may be disposed to at least partially overlap the air discharger 323 in a width direction.

The compressor installation portion 312 may be installed with a buffer member that reduces vibration transmitted from the compressor 342. The buffer member may be fixed to the protrusion.

The base 310 may include a controller installation portion 313 in which the controller 700 is installed. The controller installation portion 313 may be formed between the base bottom 311 and the circulation duct 320. The controller installation portion 313 may be formed between the base bottom 311 and a bottom surface of the circulation duct 320. The controller installation portion 313 may be formed in a duct shape in which one of a front side and a rear side is open under the circulation duct 320.

A structure of the controller installation portion 313 will be described later.

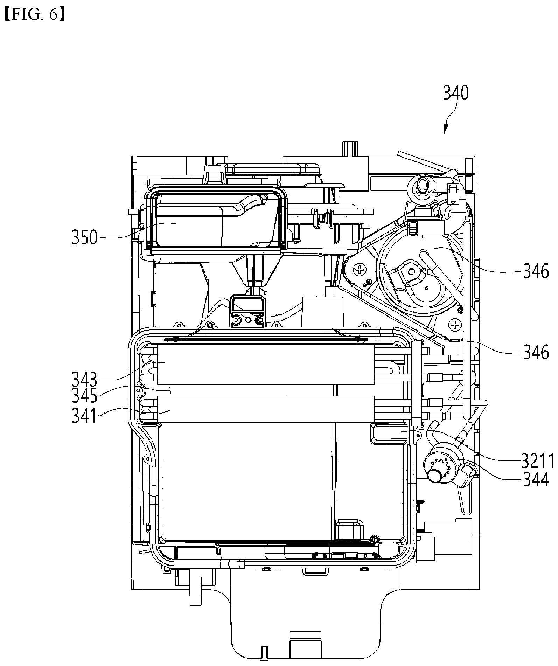

FIG. 6 shows a circulation duct structure of a laundry treating apparatus of the present disclosure.

The circulation duct 320 may extend upward from the base bottom to form the flow channel through which air flows. The circulation duct 320 may include the heat exchanger installation portion 3212 that provides the space in which the evaporator 341 and the condenser 342 are installed. The heat exchanger installation portion 3212 may be disposed inside the duct body 321.

The duct body 321 may have an open top surface. The condenser 343 and the evaporator 341 may be inserted and installed via the opening of the duct body 321.

The opening of the duct body 321 may be shielded by the base cover 360, and the base cover 360 and the duct body 321 may form the flow channel of a circulation duct 320.

The front surface of the duct body 321 may be disposed to be spaced rearwardly apart from a front end of the base bottom 311.

As a result, the base bottom 311 may secure a support surface 3111 on which at least one of the water supply tank 30 or the drainage tank 40 and the outside air duct 370 described above is installed and supported.

In one example, the heat supply 340 may include the evaporator 341 that is installed inside the circulation duct 320 and is equipped as a heat exchanger for cooling and dehumidifying air introduced into the circulation duct 320, the condenser 343 that is equipped as a heat exchanger for heating air that has passed through the evaporator 341 to generate hot air, the compressor 342 that supplies the refrigerant for heat exchange with air to the condenser 343 and is disposed outside the circulation duct 320, and an expansion valve 344 that expands and cools the refrigerant that has passed through the condenser 343.

In one example, because the duct body 321 is integrally formed with the base 310, a height of the heat exchanger installation portion 3212 may be secured more, and heights of the condenser 343 and the evaporator 341 may also be increased.

As a result, widths in the front and rear direction of the condenser 343 and the evaporator 341 may be reduced, so that the number of refrigerant pipes passing through the condenser and the evaporator may be reduced. Accordingly, there is an effect of reducing a flow loss of air passing through the condenser and the evaporator.

In one example, a sum of a length of the evaporator 341 and a length of the condenser 343 may be smaller than a length of the heat exchanger installation portion 3212. Accordingly, the length in the front and rear direction of the heat exchanger installation portion 3212 may be equal to or smaller than half of a length of the duct body 321.

Therefore, because the heat exchanger installation portion 3212 may be sufficiently spaced apart from the outside air intake portion 322, sufficient space for outside air and air inside the inner casing 200 to be introduced into the circulation duct 320 may be secured.

In one example, the inside of the duct body 321 may include an installation partition wall 3211 that separates the heat exchanger installation portion 3212 from the outside of the heat exchanger installation portion 3212. The installation partition wall 3211 may protrude from a side surface of the duct body 321 and support a front side of the evaporator 341.

In addition, the duct body 321 may extend rearward with a width expanded based on the installation partition wall 3211.

As a result, a width of the heat exchanger installation portion 3212 may be greater than half the width of the base 310. In addition, a width of the circulation duct 320 may be greater than half the width of the base 310.

The width of the condenser 343 and the width of the evaporator 341 may also be greater than half the total width of the base 310.

As described above, when the widths of the condenser 343 and the evaporator 341 are secured, a heat exchange capacity may be sufficiently secured.

In addition, the fan housing 350 may be disposed to overlap the condenser 343 or the evaporator 341 in the front and rear direction. Therefore, air that has passed through the evaporator 341 and the condenser 343 may be introduced into the fan housing 350 without bending of the flow channel. In other words, air introduced into the circulation duct 320 may minimize the flow loss because the flow channel is not bent during the process of flowing to the fan housing.

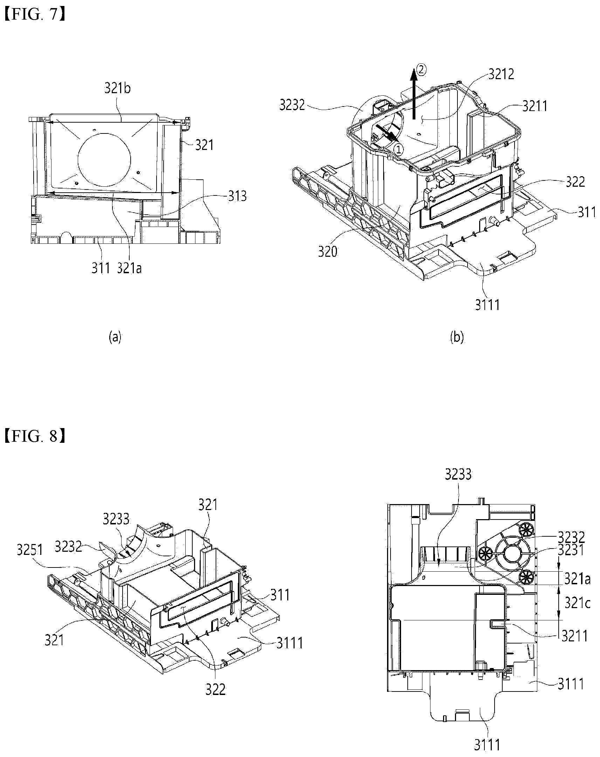

FIG. 7 illustrates a shape of a circulation duct of a laundry treating apparatus of the present disclosure.

The base 310 may have the base bottom 311 and the circulation duct 320 formed integrally with each other via mold injection.

A mold forming an inner surface of the duct body 321 may be removed by being withdrawn upward from inside of the duct body 321. In this regard, to facilitate the withdrawal of the mold, a wall surface of the duct body 321 may be inclined at a predetermined angle with respect to the removal direction of the mold.

A width of a bottom surface 321a of the duct body 321 may be greater than a width of a top surface 321b of the duct body 321.

Specifically, a distance between the wall surfaces of the duct body 321 facing each other may increase as it gets farther from the base bottom 311. A distance between a left side surface and a right side surface of the circulation duct facing each other may increase along the withdrawal direction of the mold. Accordingly, the removal of the mold may become easier.

In one example, the air discharger 323 may include an air extension pipe 3231 that extends from a rear side of the duct body 321 such that a diameter or a width thereof decreases, and an air discharge pipe 3232 that extends from the air extension pipe 3231 in a pipe shape with a uniform diameter to define a hollow 3233 therein. The air extension pipe 3231 may perform a function of a nozzle and thus increase a speed of discharged air.

In addition, a mold forming the air discharger 323 may be removed as shown in the drawing above. The mold may be withdrawn forward from the inside of the air discharger 323 toward the inside of the circulation duct 320, and then removed toward the upper open surface of the circulation duct 320. The air discharger 323 may be formed in a structure that facilitates the withdrawal of the mold in such process.

FIG. 8 is a cross-sectional view of the circulation duct.

The installation partition wall 3211 may protrude inward from the inner wall of the duct body 321 or may be formed as an outer wall of the circulation duct is recessed inward.

The heat exchanger installation portion 3212 may be formed between the heat exchanger installation partition wall 3211 and the air discharger 323.

The mold forming the air discharger 323 may be removed by being withdrawn forward from the air discharger 323 and then being withdrawn upward. It is necessary to prevent the mold for forming the above air discharger 323 from interfering with the heat exchanger installation partition wall when it is withdrawn forward from the inside of the air discharger 323. To this end, a design value of the air discharger 323 may be adjusted.

Specifically, when forming the air discharger 323, a mold for forming a front side and a mold for forming a rear side based on a parting line 3233 of the air discharger 323 in the drawing may be separately disposed. Accordingly, removal directions of the molds may also be different from each other. The mold for forming the portion located forward of the parting line of the air discharger 323 may be withdrawn forward, and the mold for forming the portion located rearward of the parting line of the air discharger 323 may be withdrawn rearward.

That is, to prevent the mold withdrawn forward from interfering with the heat exchanger installation partition wall during the withdrawal process, a distance 1 323a may be smaller than a distance 2 321c in the drawing. The distance 1 323a may mean a distance between the parting line of the air discharger 323 and a front end of the air discharger 323. In addition, the distance 1 323a may mean a distance between the parting line of the air discharger 323 and a rear opening of the circulation duct. The distance 2 321c may mean a distance between the front end of the air discharger 323 and the heat exchanger installation partition wall. In addition, the distance 2 321c may mean a distance between the rear opening of the circulation duct and the heat exchanger installation partition wall 3211.

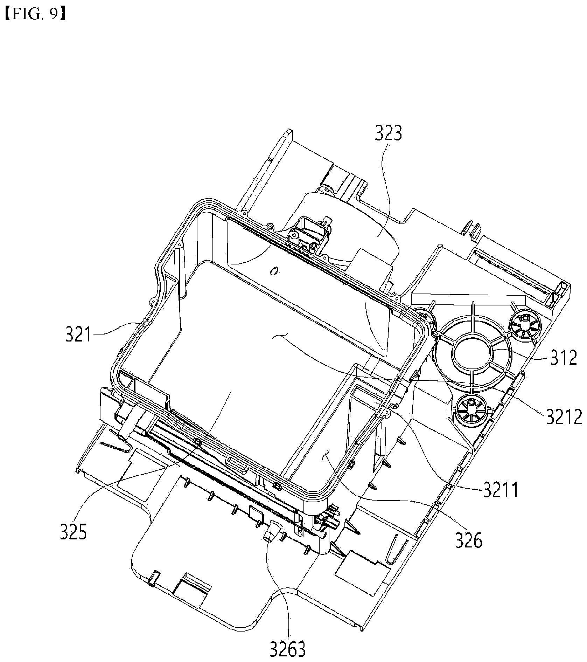

FIG. 9 shows a structure of a reservoir of a laundry treating apparatus of the present disclosure in detail.

In the laundry treating apparatus of the present disclosure, when the compressor 342 and the blower fan 352 are operated, air supplied from the outside of the cabinet 100 and air supplied from the inner casing 200 are cooled while passing through the evaporator 341, and water vapor contained in air is condensed.

Water condensed in the evaporator 341 may accumulate on the bottom surface of the circulation duct 320.

The laundry treating apparatus of the present disclosure may include a reservoir 326 formed as a portion of the bottom surface of the duct body 321 is recessed to collect condensate condensed in the evaporator 341.

The reservoir 326 is a space defined as the portion of the bottom surface of the duct body 321 is recessed, and is also able to form one side surface of the controller installation portion 313.

The reservoir 326 may be formed to be recessed downward from the bottom surface of the circulation duct 320.

The reservoir 326 may be formed integrally with the circulation duct 320. The reservoir 326 may be formed by forming the portion of the bottom surface of the circulation duct 320 to be recessed when injection-molding the circulation duct 320 onto the base 310.

At least a portion of a top surface of the reservoir 326 may be disposed parallel to the heat exchanger installation portion 313.

The base 310 may include a drain pipe 3263 that discharges water collected in the reservoir 326 to the outside.

The drain pipe 3263 may protrude from a lower portion of the reservoir 326 to the outside of the circulation duct 320. The drain pipe 3263 may discharge water stored in the reservoir to the outside of the base. As a result, water collected in the reservoir 326 may be prevented from rotting or flowing back to the bottom surface of the circulation duct 320.

The circulation duct 320 has the partition wall 3211 that extends from an inner surface of the duct body 321. The partition wall 3211 may protrude inward from the inner wall of the circulation duct 320 or may protrude inward as an outer wall of the circulation duct 320 is recessed inward. The partition wall 3211 may guide the locations where the heat exchangers 341 and 343 are installed, and may prevent air entering the heat exchanger from bypassing the heat exchanger.

The partition wall 3211 may be disposed in the reservoir 326.

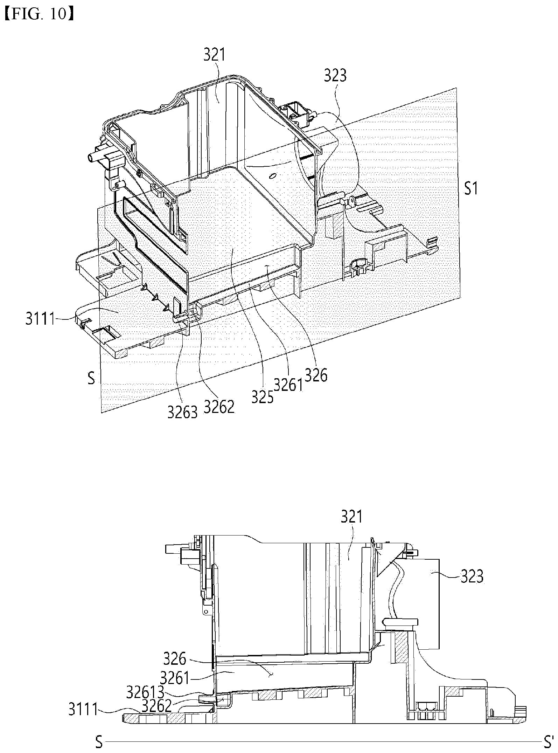

FIG. 10 is a cross-sectional view (S-S') of a circulation duct cut in a height direction.

The reservoir 326 may include a bottom surface 3261 where water accumulates, and a recessed portion 3262 that is recessed further downward from the bottom surface 3261. The drain pipe 3263 may be disposed on an outer surface of the circulation duct 320 at a location corresponding to the recessed portion 3262. As a result, the drain pipe 3263 may be disposed at a portion of the reservoir 326 with the lowest water level. Water collected in the reservoir 326 may flow to the drain pipe 3263 by its own weight.

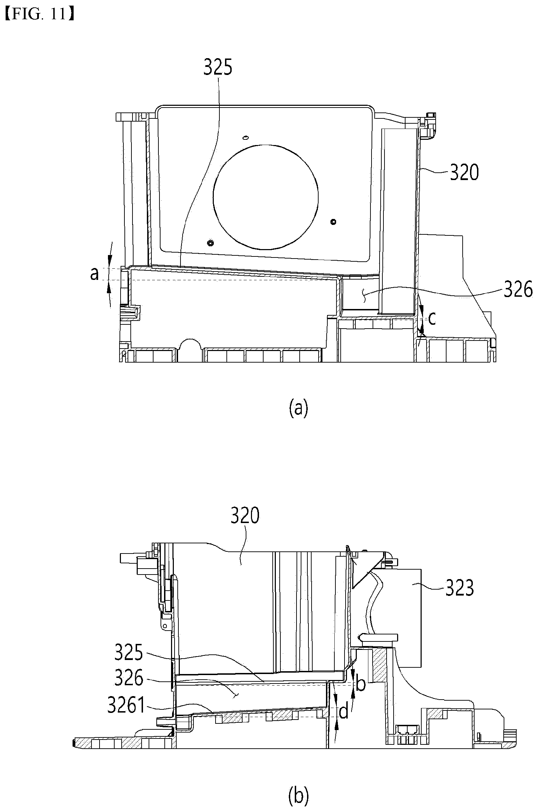

FIG. 11 shows an inclined structure related to a reservoir.

(a) in FIG. 11 shows a vertical cross-section parallel to the width direction of the base, and (b) in FIG. 11 shows a vertical cross-section parallel to the front and rear directions of the base.

The bottom surface of the circulation duct 320 and the bottom surface of the reservoir 326 may have a predetermined inclination.

In particular, a bottom surface 325 of the circulation duct may be inclined toward the reservoir 326, and the bottom surface 3261 of the reservoir may be inclined toward the drain pipe 3263.

The bottom surface 325 of the circulation duct may be disposed to be inclined at an angle 1 a toward the reservoir 326 based on the ground or the bottom surface of the base 310.

In addition, the bottom surface 325 of the circulation duct may be inclined downward in a forward direction toward the drain pipe 3263. The bottom surface 325 of the circulation duct may be disposed to be inclined forward at an angle 2 b based on the bottom surface of the base 310.

As a result, water condensed on the bottom surface of the circulation duct may flow forward and toward the reservoir 326.

In one example, the bottom surface 3261 of the reservoir may also have a predetermined inclination.

The drain pipe 3263 may be disposed to be biased on an inner surface of the reservoir 326 rather than on the outer surface thereof.

The bottom surface 3261 of the reservoir may have a downward inclination toward the inside of the circulation duct 320 based on the bottom surface of the base 310.

The bottom surface 3261 of the reservoir may be inclined at an angle 3 c based on the bottom surface of the base 310, and the bottom surface 3261 of the reservoir may have an inclination direction opposite to the inclination of the bottom surface 325 of the circulation duct.

The angle 3 c may be an angle of downward inclination in a direction away from the partition wall 3211.

The bottom surface 3261 of the reservoir may have a downward inclination toward the drain pipe 3263.

The bottom surface 3261 of the reservoir 326 may have a downward inclination in a forward direction at an angle 4 d based on the base 310.

The above-described angles 1 to 4 may be determined by a mold during a molding process of the base 310. The angles 1 to 4 may be determined during a molding process of the circulation duct 320 or the reservoir 326. The angles 2 b and 4 d may form inclinations in the same direction.

A mold for molding the reservoir 326 may be removed by being withdrawn upward. In this regard, side walls of the reservoir 326 may be tapered to facilitate the removal of the mold. Specifically, a cross-sectional area of the reservoir 326 may increase based on the withdrawal direction of the mold. In other words, a perimeter of the top surface of the reservoir 326 may be greater than a perimeter of the bottom surface of the reservoir 326.

A front surface of the reservoir 326 may be inclined forward in an upward direction. A rear surface of the reservoir 326 may be inclined rearward in the upward direction. Left and right side surfaces of the reservoir 326 may be inclined leftward and rightward in the upward direction, respectively.

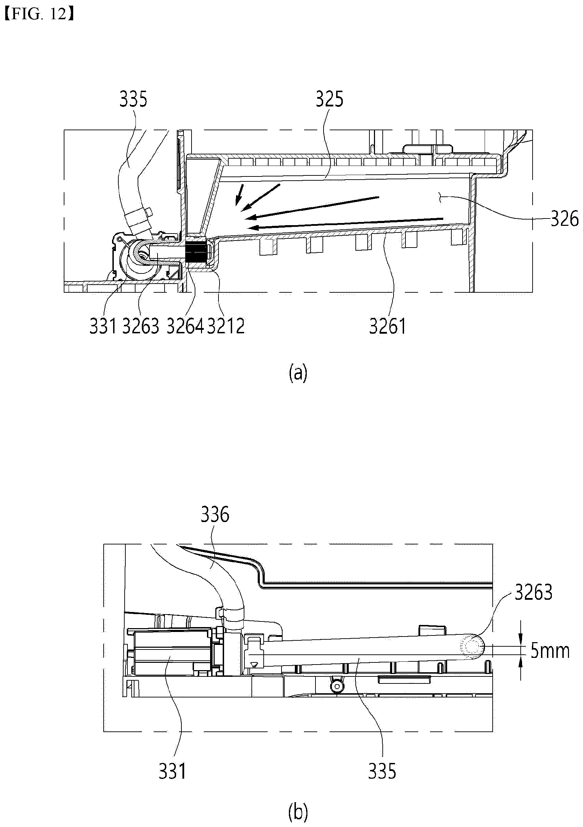

FIG. 12 shows structures of the reservoir and a residual water treater.

(a) in FIG. 12 is a cross-sectional view in a front and rear direction of the reservoir, and (b) in FIG. 12 shows a front portion of the bottom surface of the circulation duct 320.

The reservoir 326 may be disposed such that the bottom surface 3261 is inclined downward in a forward direction, and the bottom surface 325 of the circulation duct 320 may also be inclined downward in the forward direction.

A filter 3264 may be disposed in the recessed portion 3262 to prevent foreign substances from being discharged outside the drain pipe 3263.

The laundry treating apparatus of the present disclosure may include a residual water treater 330 that collects water collected in the reservoir 326 into the drainage tank 40.

The residual water treater 330 may include a drainage pump 331 that discharges water collected in the reservoir 326 into the drainage tank 40. The drain pipe 3263 and the drainage pump 331 may be connected to each other via a first drainage hose 3351, and water discharged from the drainage pump 331 may flow along a second drainage hose 3352.

The drain pipe 3263 may be disposed upward of the drainage pump 331. As a result, water collected in the reservoir 326 by its own weight may be collected to the drainage pump 331.

Water collected to the drainage pump 331 may be collected in the drainage tank 40 when the drainage pump 331 operates.

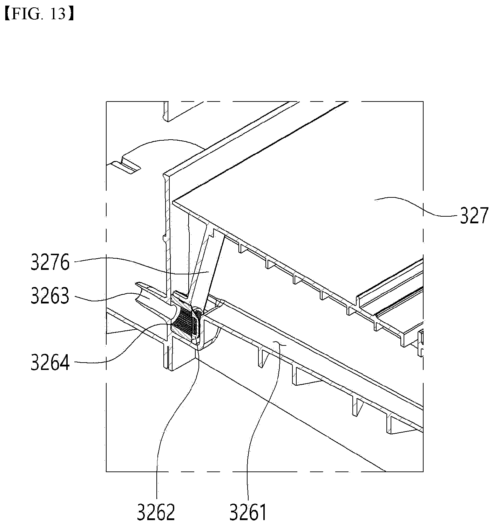

FIG. 13 shows an embodiment of a water cover.

The laundry treating apparatus of the present disclosure may further include a water cover 327 that is seated on the bottom surface of the circulation duct 320. The water cover 327 may support at least one of the evaporator 341 and the condenser 343, may block water condensed in the evaporator 341 from flowing to the condenser 343, and guide water to the reservoir 326.

The water cover may prevent the bottom surface of the circulation duct from being exposed to the outside. In addition, the water cover may form the support surface on which the evaporator and the condenser are supported. The water cover may support the evaporator and the condenser so as to be spaced apart from the bottom surface of the circulation duct. The water cover may shield the top surface of the reservoir. That is, the water cover may perform a cover function of the reservoir.

The water cover 327 may also shield an upper portion of the reservoir 326. As a result, air introduced into the circulation duct 320 may be blocked from being obstructed by a step between the reservoir 326 and the circulation duct 320.

The water cover 327 may include a water body 3271 having a plate shape and supporting at least one of the evaporator 341 and the condenser 343, and a support rib 3276 extending downward from the water body 3271 to maintain a vertical level or an inclination of the water body 3271.

One of the support ribs 3276 may be supported by the recessed portion 3262 or the filter 3264. As a result, the support rib 3276 may directly guide water flowing along the water body 3271 to the drain pipe 3263.

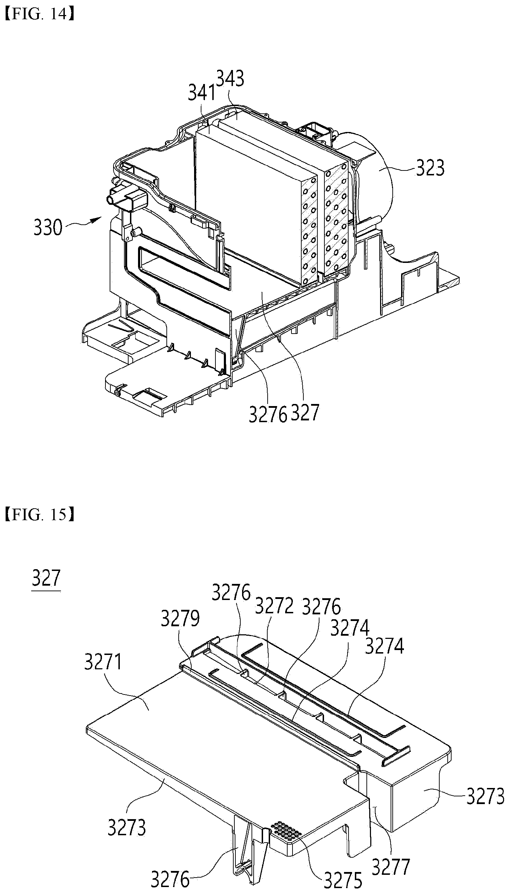

FIG. 14 shows a state in which the water cover is installed in a circulation duct.

The water cover 327 may be formed in a plate shape that shields at least a portion of the bottom surface of the circulation duct 320.

The water cover 327 may block the reservoir 326 from being exposed to an area facing the inlet 362 or an area into which outside air is introduced.

The water cover 327 may support lower ends of the evaporator 341 and the condenser 343. Even when the bottom surface of the circulation duct 320 is disposed to be inclined because of the water cover 327, the evaporator 341 and the condenser 343 may be disposed at the same vertical level.

In addition, the water cover 327 may prevent locations of the evaporator 341 and the condenser 343 from changing.

The water body 3271 of the water cover 327 may be disposed with an inclination to be parallel to the base 310. This prevents air introduced into the evaporator 341 from receiving unnecessary inclination resistance.

FIG. 15 shows a detailed structure of a water cover.

The water cover may include the water body 3271 positioned upward of the bottom surface of the circulation duct or the bottom surface of the reservoir. The water body 3271 may prevent the bottom surface 325 of the circulation duct or the bottom surface 3261 of the reservoir from being exposed to the outside.

The water cover 327 may include a seating rib 3274 protruding upward from the water body 3271. The seating rib 3274 may fix at least one of the evaporator 341 and the condenser 343, and may also maintain a gap between the evaporator 341 and the condenser 343.

The water cover 327 may include a through-hole 3272 extending through the water body 3271. The through-hole 3272 may be defined between the evaporator 341 and the condenser 343. Thus, water condensed in the evaporator 341 may be guided downward of the water cover.

The water cover 327 may further include a water outlet 3275 extending through the water body 3271 and defined to be spaced apart from the through-hole 3272. The water outlet 3275 may be defined in an area facing the reservoir 326.

The water outlet 3275 may discharge water flowing along the top surface of the water body 3271 to the reservoir 326.

In addition, the water outlet 3275 may also guide water overflowing from the drainage tank 40 to the reservoir 326.

The water cover 327 may include a spacing rib 3273 supported on the bottom surface of the circulation duct 320 from the water body 3271. The spacing rib 3273 may have an inclination corresponding to the inclination of the bottom surface of the circulation duct 320, and may be in contact with the bottom surface of the circulation duct 320, thereby blocking the air from flowing between the water body 3271 and the bottom surface of the circulation duct 320.

In one example, the spacing rib 3273 may be disposed along a periphery of the water body 3271.

In one example, the water cover 327 may further include an avoidance portion 3277 that prevents interference with the partition wall 3211 of the circulation duct. The avoidance portion 3277 may be formed to be recessed from a side surface of the water body 3271. The avoidance portion 3277 may have a shape corresponding to a shape of the partition wall.

The water cover 327 may include a discharge rib 3276 supported by the reservoir 326. The discharge rib 3276 may be formed in a shape that does not shield the drain pipe 3263.

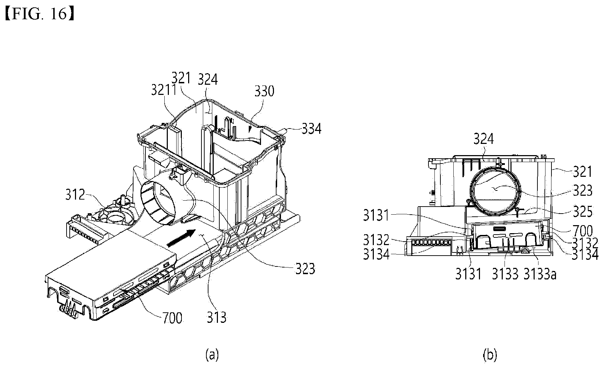

FIG. 16 shows a structure of a controller installation portion disposed on a base of a laundry treating apparatus of the present disclosure.

(a) in FIG. 16 shows an aspect in which the controller 700 is installed in the controller installation portion 313.

The controller 700 may control all devices necessary for the laundry treating apparatus of the present disclosure to perform an arbitrary course of performing the refreshing cycle of the laundry. The controller 700 may be equipped as a PCB board, but may not be limited thereto and may be equipped as various devices for control.

The controller 700 may be inserted into and seated in the controller installation portion 313.

The controller installation portion 313 may be disposed downward of the circulation duct 320.

The bottom surface of the circulation duct 320 may form a top surface of the controller installation portion 313. The controller installation portion 313 may be disposed downward of the air discharger 323.

The controller installation portion 313 may be formed integrally with the base bottom 311. The controller installation portion 313 may be defined as a recessed space beneath the circulation duct during the process of forming the circulation duct 320 on the base 310.

The controller 700 may be introduced in a sliding manner in the forward direction into the controller installation portion 313.

A bracket 3131 may be further disposed on a surface of the controller 700 to surround the controller. The brackets 3131 may be disposed top and bottom surfaces of the controller to prevent the foreign substances from entering the controller.

In addition, the bracket 3131 may prevent damage to a circuit board inside the controller 700 resulted from heat or vibration transmitted to the controller 700. The bracket 3131 may be made of a metal material.

(b) in FIG. 16 shows a state in which a controller is installed in a controller installation portion.

As shown in the drawing, the controller 700 may be installed at a predetermined angle with the base bottom 311.

For example, the controller 700 may be disposed to be inclined toward the reservoir 326. Accordingly, when water leaks onto the top surface of the controller 700, the water may quickly escape the controller 700, and the bottom surface of the circulation duct 320 may be formed to be inclined toward the reservoir 326.

The controller 700 may include a supporter 3132 that is formed to protrude laterally.

The controller installation portion 313 may include a rib 3134 that protrudes from each of both side surfaces of the installation portion. The supporter 3132 of the controller may be disposed on the rib 3134.

The supporter 3132 of the controller may support a load of an entirety of the controller 700. When the supporter 3132 of the controller is supported on the rib 3134, the controller 700 may be spaced apart from the base bottom 311 by a predetermined distance.

The rib 3134 may be formed integrally with the base 310. The rib 3134 may be formed together with the base 310 when the base 310 is injection-molded, and may be formed integrally with the base bottom 311, the circulation duct 320, and the like.

A protrusion 3133 may be formed to protrude on a front surface of the controller 700. In addition, a guide protruding rearward may be disposed on an inner surface of the controller installation portion 313. The protrusion may be coupled with the guide. The protrusion may be inserted into the guide. When inserting the controller into the controller installation portion, the controller may be aligned at a correct location by coupling the protrusion to the guide.

In addition, locations of both side surfaces of the controller may be determined in the manner in which the supporter is seated on the rib as described above. Using such coupling process, the controller may be coupled at the correct location of the controller installation portion without a separate fastening member.

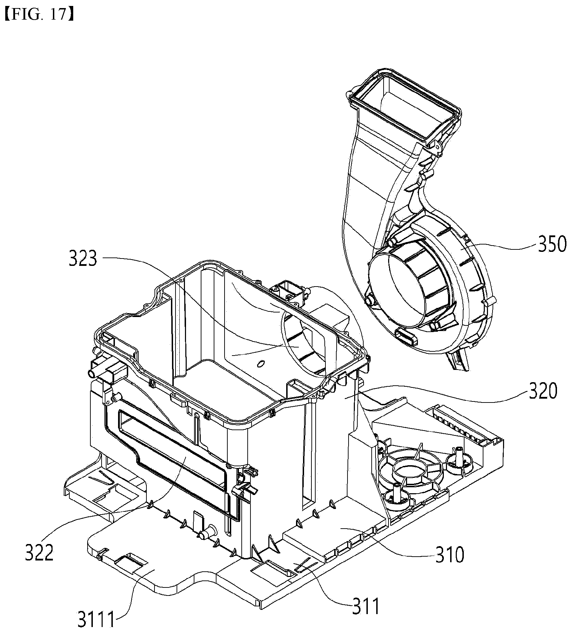

FIG. 17 shows a structure of an air discharger 323 of a laundry treating apparatus of the present disclosure.

The base 310 may include the air discharger 323 that discharges treated air toward the fan housing.

The air discharger 323 may allow the inside of the circulation duct 320 or the duct body 321 to be in communication with the fan housing 350. The air discharger 323 may be formed in a bell mouth shape. The bell mouth shape may reduce the flow loss of air and improve an air circulation efficiency.

The air discharge pipe 3232 of the air discharger 323 may be formed in a pipe shape, and in the mold removal process, based on the parting line 3233, the mold disposed forward of the parting line 3233 may be withdrawn forward, and the mold disposed rearward of the parting line 3233 may be withdrawn rearward.

The fan installation portion 350 may be supported by being coupled to the air discharge pipe 3232. The fan housing 351 may have a coupling hole coupled with an outer circumferential surface of the air discharge pipe 3232, and the blower fan 353 may be disposed in the coupling hole.

The fan housing 351 may include the exhaust duct 352 extending from an outer circumferential surface or an outer side of the blower fan 353 to the exhaust hole 232.

The fan housing 351 and the exhaust duct 352 may form therein a flow channel that accommodates the blower fan 353 therein and allows air to flow.

A motor that rotates the blower fan 353 may be supported by being coupled to the outer side of the fan housing 351.

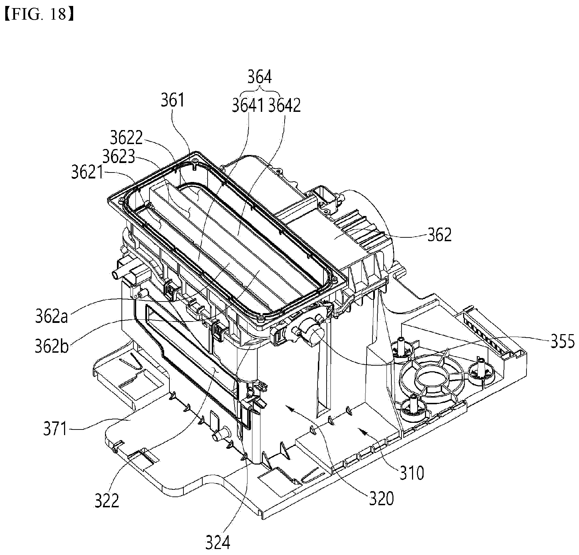

FIG. 18 shows a structure of a base cover of a laundry treating apparatus of the present disclosure.

The base cover 360 may be coupled to the top surface of the circulation duct 320 to prevent the inside of the circulation duct 320 from being exposed.

The base cover 360 may include an inflow body 361 coupled to the top surface of the circulation duct 320 to allow the inner casing 200 and the circulation duct 320 to be in communication with each other, and a shielding body 363 extending from the inflow body 361 to shield the circulation duct 320.

The inflow body 361 may be formed in a duct shape to allow the inflow hole 231 of the inner casing and the inside of the circulation duct 320 to be in communication with each other. The inflow body 361 may protrude higher than the shielding body 363.

The inflow body 361 may be disposed forward of the evaporator 341 so as not to face the evaporator 341 and the condenser 343, and may be disposed forward of the partition wall 3211.

The inflow body 361 may serve as an inflow duct that moves air of the inner casing 200 to the circulation duct 320.

The inflow body 361 may include the inlet 362 therein through which air of the inner casing 200 may pass.

Specifically, the base cover 360 may include a first rib 362a extending along a width direction of the inflow body 361 and a second rib 362b extending along the width direction of the inflow body 361 and spaced rearwardly apart from the first rib 362a.

The first rib 362a and the second rib 362b may be disposed to be in parallel with each other. The first rib 362a and the second rib 362b may be formed in a plate shape extending in a vertical direction, and a height thereof may correspond to a height of the inflow body 361.

A front edge of the inflow body 361 and the first rib 362a may form a first inlet 3621, the first rib 362a and the second rib 362b may form a second inlet 3622, and the second rib 362b and a rear edge of the inflow body 361 may form a third inlet 3623.

The first inlet 3621 and the third inlet 3623 may have the same area size, and the second inlet 3622 may have a smaller area size than the first inlet 3621 and the third inlet 3623.

The base cover 360 may include a damper assembly 364 that opens and closes the inlet 362, and a driver 365 coupled to the damper 364 to control the opening and closing of the damper assembly 364.

The damper assembly 364 may include a first damper 3641 that opens and closes the first inlet 3621, and a second damper 3642 that opens and closes the third inlet 3623.

The first damper 3641 may be formed in a plate shape with an area size corresponding to that of the first inlet 3621, and may be rotatably coupled to both side surfaces of the inflow body 361 inside the first inlet 3621.

The second damper 3642 may be formed in a plate shape with an area size corresponding to that of the third inlet 3623, and may be rotatably coupled to both side surfaces of the inflow body 361 inside the third inlet 3623.

The second inlet 3622 may include a blocking filter 366 that allows air to pass therethrough, but is able to filter out the foreign substances such as fine dust and lint.

The blocking filter 366 may be inserted into the second inlet 3622 to partition the first inlet 3621 and the third inlet 3623 from each other. The blocking filter 366 may be disposed in the second inlet 3622 to extend so as to be in contact with the bottom surface of the circulation duct 320.

The blocking filter 366 may be equipped as a filter that may filter even moisture. For example, the blocking filter 366 may be equipped as a HEPA filter or the like.

In one example, when the blocking filter 366 is inserted into the second inlet 3622, a shielding member that shields the second inlet 3622 may be further coupled.

The driver 365 may include a motor that provides power to selectively rotate the first damper 364 and the second damper 365, and a plurality of gear members that may selectively rotate the first damper 364 and the second damper 365 while rotating in a state of being engaged with the motor.

The first inlet 3621 and the third inlet 3623 may be selectively opened by the driver 365.

Because of the driver 365, air contained within the inner casing 200 may be introduced into the circulation duct 320 along the first inlet 3621 or may be introduced into the circulation duct 320 along the third inlet 3623.

In one example, the driver 365 may control the first damper 3641 and the second damper 3642 to open both the first inlet 3621 and the third inlet 3623, and may control the first damper 3641 and the second damper 3642 to shield both the first inlet 3621 and the third inlet 3623.

The driver 365 may be formed with any structure as long as it may rotate the first damper 3641 and the second damper 3642. For example, the driver 365 may be formed with a combination of the motor, a driving gear that rotates by the motor, and a driven gear that is coupled to the first damper and the second damper and rotates by the rotation of the driving gear.

The base cover 360 may include the shielding body 363 that may extend from the inflow body 361 and shield the evaporator 341 and the condenser 343. The shielding body 363 may be formed in a plate shape.

The base cover 360 may be detachably coupled to the top surface of the circulation duct 320 via an inflow hook 3612 that extends from the bottom surface of the inflow body 361.

The circulation duct 320 may include a coupling portion that is detachably coupled to the inflow hook 3612.

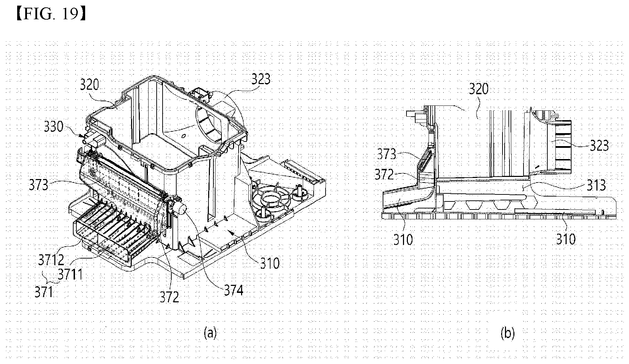

FIG. 19 shows a structure of an outside air duct.

Referring to (a) in FIG. 19, the outside air duct 370 may be coupled to the base 310.

The outside air duct 370 may be in communication with the outside air intake portion 322.

The outside air duct 370 may include an outside air damper 373 that opens and closes the outside air intake portion 322, and an outside air driver 374 that rotates the outside air damper 373 to selectively open the outside air intake portion 322.

The outside air damper 373 may be formed in a plate shape that may seal the outside air intake portion 322, and may be rotatably coupled to both side surfaces of the outside air intake portion 322.

The outside air driver 374 may be equipped as an actuator that is coupled to the outside air duct 370 or the circulation duct 320 and rotates the outside air damper 373.

The outside air duct 370 may include an extension duct 372 that extends forward from the outside air intake portion 322, and an intake duct 371 that extends forward from the extension duct 372 and allows outside air to be introduced.

The intake duct 371 may extending from a lower portion of the extension duct 372, and may have the water supply tank 30 and the drainage tank 40 disposed thereon. The water supply tank 30 and the drainage tank 40 may be coupled to or seated on the intake duct 371.

The intake duct 371 may include an outside air port 3711 into which outside air is sucked and a partition rib 3712 that partitions the outside air port 3711 at one end or a free end thereof.

The outside air port 3711 may be disposed downward of the door 400 so as not to be shielded by the door 400.

The partition rib 3712 may partition the inside of the outside air port 3711 so as to block the foreign substances or a user's body from being inserted.

Referring to (b) in FIG. 19, when the outside air driver 374 rotates the outside air damper 373 to open the outside air intake portion 322, the intake duct 371 and the circulation duct 320 may be in communication with each other.

In this regard, when the blower fan 352 is operated, air outside the cabinet may be introduced into the circulation duct 320. When the compressor 342 is operated, the outside air may be dehumidified while passing through the circulation duct 320 and supplied into the inner casing 200.

The door 400 may further include an exhaust port that discharges air inside the inner casing 200 to the outside, and an exhaust damper that selectively opens and closes the exhaust port. The exhaust port may be disposed to face the accommodating space of the inner casing 200.

Thus, the dehumidified air may be discharged via the exhaust port.

In addition, the outside air may be filtered while passing through the blocking filter 366 and discharged back to the outside of the cabinet 100.

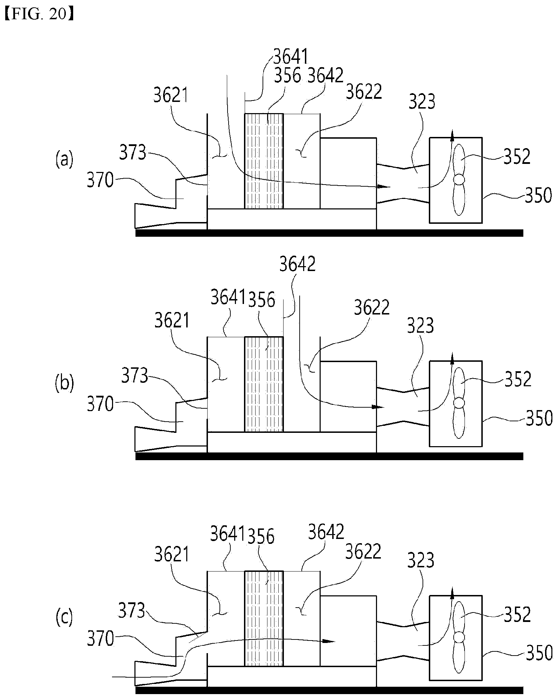

FIG. 20 shows a flow of air flowing through the circulation duct.

Referring to (a) in FIG. 20, the outside air damper 373 may be controlled to shield the outside air intake portion 322, the first damper 3641 may be controlled to open the first inlet 3621, and the second damper 3642 may be controlled to shield the third inlet 3623.

When the blower fan 352 is operated, air inside the inner casing 200 may be introduced into the first inlet 3621 and filtered while passing through the filter 366.

When the compressor 342 is operated, air that has passed through the filter 366 may be dehumidified and heated while passing through the evaporator 341 and the condenser 343.

Air that has also passed through the heat exchanger may be supplied into the inner casing 200 by passing through the fan installation portion 350.

This state may be a state in which steam is not supplied to the inner casing 200. This is because when steam is supplied to the inner casing 200, moisture will wet the filter 600, and a performance of the filter 366 will not be guaranteed.

As a result, when steam has not been supplied to the inner casing 200, before steam is supplied to the inner casing 200, or when humidity is lowered even after steam is supplied to the inner casing 200, air inside the inner casing 200 may pass through the first inlet 3641 and may filter out the foreign substances such as lint while passing through the filter 366.

Referring to (b) in FIG. 20, the outside air damper 373 may be controlled to shield the outside air intake portion 322, the first damper 3641 may be controlled to shield the first inlet 3621, and the second damper 3642 may be controlled to open the third inlet 3623.

When the blower fan 352 is operated, air inside the inner casing 200 may be introduced into the third inlet 3623. Because the third inlet 3623 is disposed downstream of the blocking filter 366, air introduced into the third inlet 3623 may not pass through the blocking filter 366.

When the compressor 342 is operated, air that has passed through the filter 366 may be dehumidified and heated while passing through the evaporator 341 and the condenser 343.

Air that has also passed through the heat exchanger may be supplied into the inner casing 200 by passing through the fan installation portion 350.

As a result, when steam has been supplied to the inner casing 200 or the humidity inside the inner casing 200 is very high, air of the inner casing 200 may be allowed to be introduced into the third inlet 3623 and blocked from being introduced into the first inlet 3621, thereby preventing the blocking filter 366 from being exposed to moisture.

Referring to (c) in FIG. 20, the outside air damper 373 may be controlled to open the outside air intake portion 322, the first damper 3641 may be controlled to shield the first inlet 3621, and the second damper 3642 may be controlled to shield the third inlet 3623.

When the blower fan 352 is operated, air inside the inner casing 200 may be blocked from flowing into the inlet 362, and only outside air of the cabinet 100 may flow into the circulation duct 320 and pass through the blocking filter 366. As a result, the foreign substances such as fine dust contained in outside air may be filtered by the blocking filter 366.

When the compressor 342 is operated, air that has passed through the filter 366 may be dehumidified while passing through the evaporator 341 and the condenser 343.

Air that has also passed through the heat exchanger may be supplied into the inner casing 200 by passing through the fan installation portion 350 to supply fresh hot air to the laundry.

In this regard, when an apparatus for discharging air inside the inner casing 200 to the outside is disposed in the door 400, air outside the cabinet may be discharged in a purified and dehumidified state while passing through the blocking filter 366 and the heat supply 340.

As a result, the laundry treating apparatus of the present disclosure may determine flow directions of air inside the inner casing 200 and air outside the cabinet as the controller 700 controls the outside air driver 374 and the inlet driver 365.

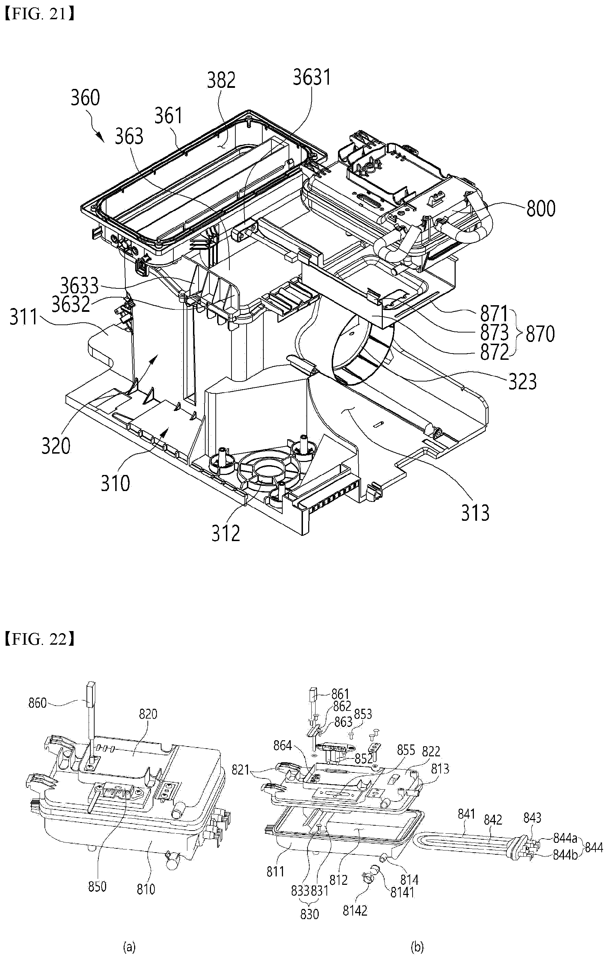

FIG. 21 shows an installation structure of a steam supply.

The steam supply 800 may be supported by being seated on the base cover 360.

The steam supply 800 may include a steam generator 810 that is seated on the base cover 360 and stores water that generates steam.

The steam supply 800 may further include an installation bracket 870 that may fix the steam generator 810 to the base cover 360.

The installation bracket 870 may be coupled to the base cover 360 to fix the steam generator 810.

The installation bracket 870 may include a lower panel 871 that supports a bottom surface of the steam generator 810 and side panels 872 that support both side surfaces of the steam generator 810 on the lower panel 871.

The installation bracket 870 may further include one or more fixing clips 873 that extend from the side panels 872 to prevent the steam generator 810 from being detached.

The fixing clip 873 may be detachably disposed on a top surface or the side surface of the steam generator 810.

The compressor 342 may be disposed downward of the steam supply 800.

The installation bracket 870 may block heat generated from the compressor or heat generated from a refrigerant compressed in the compressor from being transferred to the steam supply 800.

The installation bracket 870 may also block fire from being transferred to the steam supply 800 in the event of fire occurring in the compressor 342.

In one example, the base cover 360 may include a fastener 3631 disposed on the shielding body 363 and detachably coupled with the steam supply 800. The fastener 3631 may have a structure detachably coupled with a protrusion protruding from a lower portion of the steam generator 810.

As a result, even when a large amount of water is contained inside the steam generator 810, the steam generator 810 may be stably seated on the base cover 360.

In addition, because the steam generator 810 is positioned upward of the circulation duct 320 and thus a distance thereof to the inner casing 200 becomes smaller, condensation of steam generated in the steam generator 810 before reaching the inner casing 200 may be minimized.

FIG. 22 shows a detailed structure of the steam supply.

Referring to (a) in FIG. 22, the steam supply 800 may include a steam generator 810 that may receive water to generate steam and store water therein, and a heater assembly 840 that is accommodated in the steam generator 810 and heats the water to generate steam.

The steam generator 810 may be formed in a casing shape that defines a space for accommodating the heater assembly 840.

For example, the steam generator 810 may be formed in a casing shape with an open top, and may accommodate the heater assembly 840 therein.

The steam supply 800 may further include a casing cover 820 that is coupled to the steam generator 810 to prevent the heater assembly 840 from being exposed to the outside and prevent water from leaking.

The casing cover 820 may be installed with a water level sensor 850 that senses a water level of the steam generator 810 and a steam sensor 860 that senses a temperature inside the steam generator 810 or senses whether steam is generated inside the steam generator 810.

Referring to (b) in FIG. 22, the steam generator 810 may include a casing body 811 that provides a space for storing the water and accommodating the heater assembly 840 therein.

The casing body 811 may be formed in a shape with an open top, so that various components may be easily installed inside the casing body 811.

The casing body 811 may include a heater insertion hole 8111 extending through one side thereof and through which the heater assembly 840 may be inserted or withdrawn.

The casing body 811 may include a recovery pipe 814 that receives water to generate steam therein.

The recovery pipe 814 may discharge water accommodated inside the casing body 811 to the outside.

The recovery pipe 814 may be kept closed by a shielding plug 8141 so as to be opened only when removing residual water inside the steam generator 810, and may include a shielding clip 8142 that keeps the shielding plug 8141 coupled to the recovery pipe 814 such that the shielding plug 8141 is prevented from being removed arbitrarily.

Therefore, when repairing the steam generator 800 or preventing freezing or the like of the steam generator 800, water inside the steam generator 800 may be discharged via the recovery pipe 814.

In one example, the recovery pipe 814 may receive water from the water supply tank 30. Details will be described later.

In one example, a heater fixer 830 that may support or fix the heater assembly 840 may be installed inside the casing body 811. The heater fixer 830 may include a fixing clip 831 that fixes the heater assembly 840, and a clip fastening member 833 that fixes the fixing clip 831 to the casing body 811.

The fixing clip 831 may accommodate or surround at least a portion of the heater assembly 840.

In one example, the steam supply 800 may include a water supply pipe 815 that receives water. The water supply pipe 815 may be in communication with the water supply tank 30 to receive water.

The water supply pipe 815 may be disposed on the casing cover 820 or may be disposed at an upper portion of the steam generator 810. As a result, water may be prevented from flowing back via the water supply pipe 815.

The steam supply 800 may include a steam pipe 813 that discharges steam generated by the operation of the heater assembly 840 to the outside. The steam pipe 813 may also be disposed at an upper portion of the casing cover 820, so that water may be prevented from being discharged arbitrarily into the steam pipe 813.

The steam pipe 813 may be in communication with the steam hole 233 of the inner casing 200.

The casing cover 820 may include a water level sensor hole 854 in which the water level sensor may be installed.

The water level sensor 850 may include one or more contact protrusions 852 that are inserted into the water level sensor hole 854 and immersed in water to sense the water level, and a sensor body 851 that is coupled to the water level sensor hole 854 or supported by the casing cover 820 to maintain the contact protrusions 852 in a floating state inside the steam generator 810.

The sensor body 851 may be coupled to the casing cover 820 via a sensor fastening member 853.

In one example, the casing cover 820 may include an insertion hole 864 into which the steam sensor 860 may be installed. The steam sensor 860 may include a sensing device 861 that is inserted into the insertion hole 864 to sense whether steam is generated inside the steam generator 810, a support member 863 that fixes the sensing device 861 to the casing cover 820, and a coupling member 862 that couples the support member 863 to the casing cover 820.

The sensing device 861 may be equipped as a humidity sensor or a temperature sensor and may sense whether steam is generated inside the steam generator 810.

In one example, the casing cover 820 may include a cover hook 821 that extends forward to be coupled to the base cover 860.

In addition, a fixing protrusion 822 that may fix a lower portion of the inner casing 200 or a separate steam discharger 900 may also be disposed at a rear side of the casing cover 820.

The heater assembly 840 may be inserted into the heater insertion hole 8111, be accommodated in the steam generator 810, and receive power to heat water.

The heater assembly 840 may be equipped as a sheath heater or the like, and may be controlled by the controller 700 such that operation and stopping may be repeated.

The heater assembly 840 may include a first heater 841 that receives first power to heat water, and a second heater 842 that receives power greater than the first power to heat water.

As a result, the second heater 842 may heat a larger amount of water and generate more steam than the first heater 841.

The first heater 841 and the second heater 842 may consume maximum heater power allowed for the heater assembly 840 in the laundry treating apparatus in a shared manner. That is, when the first heater 841 consumes a portion of the maximum heater power, the second heater 842 may consume the remainder of the maximum heater power.

For example, when the general maximum heater power allowed for the heater assembly 840 is 1500W, the first heater 841 may consume 600W, and the second heater 842 may consume 880W. The remaining 20W may be allocated as a margin to account for an error or the like.

In one example, the heater assembly 840 may include three or more heaters. For example, the first heater 841, the second heater 842, and a third heater 843 may be included, and the first heater 841, the second heater 842, and the third heater 843 may consume the maximum heater power in the shared manner.

Hereinafter, a description will be made based on the heater assembly 840 being composed of the first heater 841 and the second heater 842.

Each of the first heater 841 and the second heater 842 may be formed as a U-shaped metal pipe.

The heater assembly 840 may include a heater sealer 843 that may fix the first heater 841 and the second heater 842 and seal the heater through-hole 8111, and may include a terminal assembly 844 that supplies current to the first heater 841 and the second heater 842.

The terminal assembly 844 may include a first terminal 844a that supplies current to the first heater 841 and a second terminal 844b that supplies current to the second heater 842.

The first heater 841 and the second heater 842 may be arranged at the same vertical level. Accordingly, the first heater 841 and the second heater 842 may heat water at the same water level to generate steam.

Therefore, the controller 700 may adjust an amount of steam generated and an amount of power consumed using both or selectively the first heater 841 and the second heater 842.

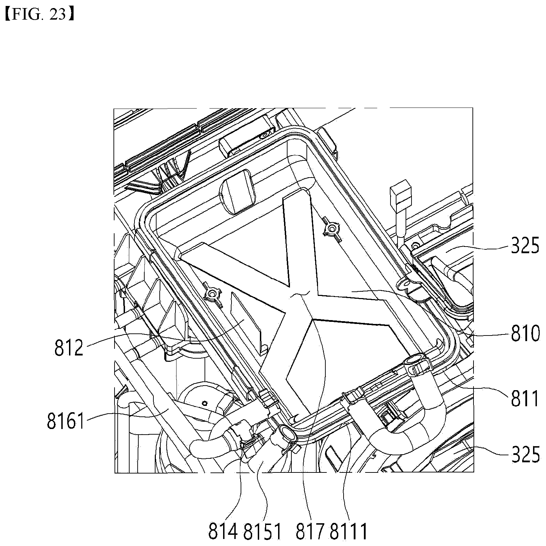

FIG. 23 shows inside of the steam generator.

The steam generator 810 may have a heating space 817 that stores water therein and accommodates the heater assembly 840 therein.

In addition, the steam generator 810 may receive water to generate steam via a water supply hose 8151 connected to the water supply pipe 815.

In one example, steam generated by operating the heater assembly 840 in the steam generator 810 may be discharged to the outside of the steam supply 800 via the steam discharge pipe 813 along a steam hose 8131.

The steam hose 8131 may be in communication with the steam hole 233 of the inner casing 200.

In one example, the steam generator 810 may include a separation partition wall 812 that may separate the heating space 817 and the water level sensor 850 from each other. That is, the separation partition wall 812 may separate the heater assembly 840 and the water level sensor 850 from each other, and may be disposed to be biased to one side of the casing body 811.

The water level sensor 850 may be disposed between the separation partition wall 812 and an inner surface of the casing body 811.

As a result, vibration of water occurring when the water boils may be prevented from being transmitted to the water level sensor 850, and heat generated in the heater assembly 840 may be prevented from being directly transmitted to the water level sensor 850.

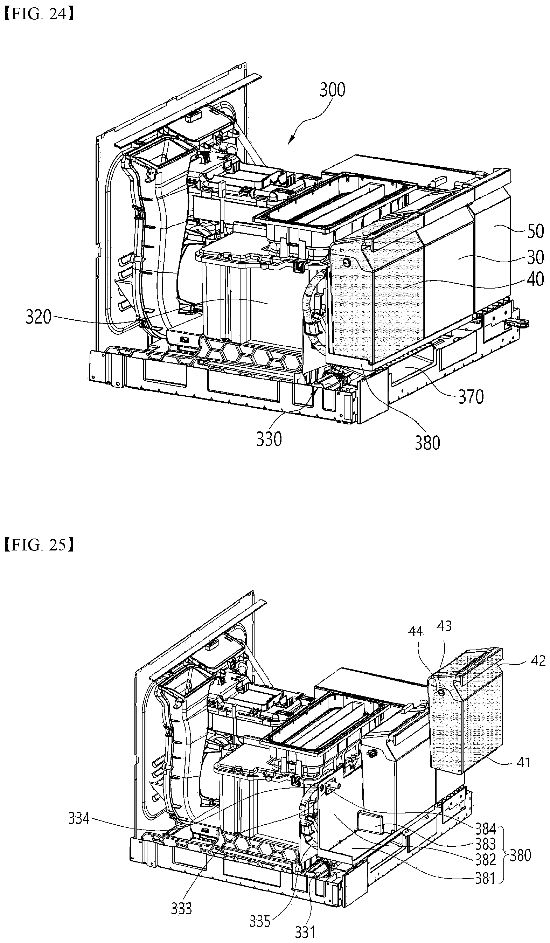

FIG. 24 shows a water tank structure of a laundry treating apparatus of the present disclosure.

The drainage tank 40 should be disposed in front of the duct body 321 so that the user may easily remove the drainage tank 40 from the machine room 300 and discharge water collected in the drainage tank 40 to the outside.

In addition, the water supply tank 30 should also be disposed in front of the duct body 321 so that the user may easily remove the water supply tank 30 from the machine room 300 and then fill the water supply tank 30 with water.

To this end, the laundry treating apparatus of the present disclosure may further include a support stand 380 that detachably supports the water supply tank 30 and the drainage tank 40 in front of the circulation duct 320.

The support stand 380 may be disposed upward of the outside air duct 370, and may support a load of at least one of the water supply tank 30, the drainage tank 40, and the drawer 50.

The support stand 380 may be fixed in front of the circulation duct 320.

The laundry treating apparatus of the present disclosure may include a residual water treater 330 that guides condensate condensed inside the circulation duct 320 to the drainage tank 40.

The residual water treater 330 may be concentratedly disposed in front of the duct body 321 to set a length of a flow channel with the drainage tank 40 to be small.

FIG. 25 shows a residual water collection structure of a laundry treating apparatus of the present disclosure.

The drainage tank 40 may include a storage body 41 that stores condensate therein, and a handle 42 that is detachably coupled to an upper portion of the storage body and supports a load of the storage body 41.

The storage body 41 may be formed in a box shape that stores water therein. The storage body 41 may include a stopper 43 that protrudes at one side thereof and is detachably fastened to the support stand 380.

The storage body 41 may include a supply hole 44 that extends through one surface to receive the condensate.

The supply hole 44 may extend through a rear surface of the storage body 41 and may be defined closer to a top surface than to a bottom surface of the storage body 41. As a result, the storage body 41 may stably store water up to a portion where the supply hole 44 is located.

The support stand 380 may include a load support 381 on which the drainage tank 40, the water supply tank 30, and the drawer 50 may be supported, and a detachment support 382 disposed on the load support 381 in front of the circulation duct 320 so that the drainage tank 40 and the water supply tank 30 may be seated.

The load support 381 may be formed in a plate shape to support bottom surfaces of the water supply tank 30 and the drainage tank 40, and the detachment support 382 may also be formed in a plate shape to support rear surfaces of the water supply tank 30 and the drainage tank 40.

The support stand 380 may include a spacer 383 protruding from the load support 381 so as to be disposed between the water supply tank 30 and the drainage tank 40. The spacer 383 may separate the water supply tank 30 and the drainage tank 40 from each other by a predetermined spacing. As a result, when one of the water supply tank 30 and the drainage tank 40 is withdrawn, the other of the water supply tank 30 and the drainage tank 40 may be prevented from being withdrawn arbitrarily.

In one example, the residual water treater 330 may include the drainage pump 331 that receives water from the drain pipe 3263 and a drainage hose 335 that discharges water from the drainage pump 331.

The drainage hose 335 may guide water supplied from the drainage pump 331 to the drainage tank 40.

In one example, the support stand 380 may include a communicating portion 384 that extends through the detachment support 382 so as to allow the drainage hose 335 and the drainage tank 40 to be in communication with each other.

The communicating portion 384 may be defined in an area facing the supply hole 44 when the drainage tank 40 is seated on the load support 381.

In one example, the residual water treater 330 of the present disclosure may further include a discharge pipe 334 that allows the drainage hose 335 and the drainage tank 40 to be in communication with each other. The discharge pipe 334 may receive water from the drainage hose 335 and guide water to the supply hole 44.

The discharge pipe 334 may extend from the circulation duct 320 or may extend from the support stand 380. The discharge pipe 334 may be formed as an injection-molded product. Accordingly, the discharge pipe 334 may stably fix a distal end of the drainage hose 335 and prevent the drainage hose 335 from being bent abruptly.

In addition, the discharge pipe 334 may be formed as an injection-molded product in a shape of a pipe that is fixed to the circulation duct 320 or the support stand 380, so that an installation location thereof may always be fixed. Accordingly, a distal end of the discharge pipe 334 and the supply hole 44 may always be positioned at correct locations, so that condensate supplied from the drainage pump 331 may be stably introduced into the drainage tank 40.

In one example, the laundry treating apparatus of the present disclosure may further include a backflow allowing portion 333 that allows water accommodated inside the drainage tank 40 to flow back into the circulation duct 320 or the residual water treater 330.

The backflow allowing portion 333 may also be installed at a location facing the supply hole 44, and may receive water inside the drainage tank 40. A detailed structure thereof will be described later.

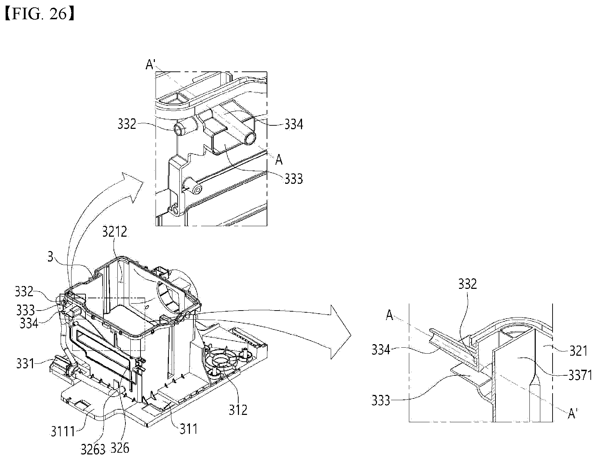

FIG. 26 shows an embodiment of a residual water treater of a laundry treating apparatus of the present disclosure.

The drain pipe 3263 may discharge condensate condensed in the reservoir 326 to the outside of the reservoir 326. The drainage pipe 3263 may extend forward from the base 310.

The drainage pump 331 may provide power to transfer water discharged via the drainage pipe 3263 to the drainage tank 40.

The drainage hose 335 may be connected to the drainage pump 331 to discharge water discharged from the drainage pump 331 to the drainage tank 40.

The drainage pump 331 and the drainage pipe 3263 should be disposed downward of the reservoir 326 to facilitate collecting condensate. Therefore, the drainage pump 331 may be disposed downward of the support stand 380.

In addition, the drainage hose 335 should be disposed at the rear of or downward of the support stand 380 to avoid hindering the drainage tank 40 from being withdrawn forward from the support stand 380.

As a result, the drainage hose 335 should be connected to the drainage pump 331 rearward of the support stand 380 and be connected to the drainage tank 40 disposed at a front side of the support stand 380 so as to supply water thereto. However, when the drainage hose 335 connects the drainage pump 331 with the drainage tank 40 at once, the drainage hose 335 should be excessively bent, so that a volume occupied by the drainage hose 335 may excessively increase, or it may be difficult to ensure durability of the drainage hose 335.

In addition, because the drainage tank 40 is disposed so as to deviate forward from the support stand 380, a separate component may be required to ensure that the drainage hose 335 and the drainage tank 40 are stably in communication with each other.

Therefore, the residual water treater 333 may further include a discharge pipe 334 that is disposed between the drainage hose 335 and the drainage tank 40 and transfers water supplied from the drainage hose 335 to the drainage tank 40.

The discharge pipe 334 may receive water transferred from the drainage hose 335 therein, but may be fixed to the support stand 380 or the circulation duct 320. The discharge pipe 334 may be disposed in an area facing the supply hole 44 when the drainage tank 40 is coupled to the detachable portion 380. Therefore, even when the drainage tank 40 is detached from or coupled to the support stand 380, the discharge pipe 334 may supply water supplied from the drainage hose 335 to the drainage tank 40.

In one example, the residual water treater 333 may further include an inlet pipe 332 connected to one side of the discharge pipe 334 and coupled with the drainage hose 335. The inlet pipe 332 may be coupled to a distal end of the drainage hose 335 and serve as a connector that changes a flow direction of water supplied from the drainage hose 335. The inlet pipe 332 may prevent the drainage hose 335 from being excessively bent.

The discharge pipe 334 may be disposed at the rear of the drainage tank 40 and may extend in the front and rear direction, and the inlet pipe 332 may be disposed to extend in a width direction from the discharge pipe 334.

The discharge pipe 334 and the inlet pipe 332 may be formed in a pipe shape that is fixed to the support stand 380 or the circulation duct 320 and supplies water.

The residual water treater 330 may install some of the components that allow the drainage pump 331 and the drainage tank 40 to be in communication with each other in the circulation duct 320. For example, the discharge pipe 334 and the inlet pipe 333 may be formed integrally with the circulation duct 320.

A flow channel that introduces condensate of the drainage tank 40 back into the circulation duct 320 and allows condensate to flow to the reservoir 326 when the drainage tank 40 is full or water contained in the drainage tank 40 overflows to the outside may be easily designed.

The residual water treater 330 may further include the backflow allowing portion 333 that introduces water contained in the drainage tank 40 into the circulation duct 320. The backflow allowing portion 333 may extend through the circulation duct 320 and be in communication with the supply hole 44 of the drainage tank 40.

To prevent water introduced into the discharge pipe 334 and the inlet pipe 332 from being introduced into the circulation duct 320 instead of flowing to the drainage tank 40, the backflow allowing portion 333 may not be in direct communication with the discharge pipe 334 and the inlet pipe 332.

The backflow allowing portion 333 may be formed adjacent to the discharge pipe 334 so as to allow water overflowing from the supply hole 44 to be re-introduced into the circulation duct 320.

The backflow allowing portion 333 may be insertable into the supply hole 44. That is, when the drainage tank 40 is seated on the support stand 380, the backflow allowing portion 333 may be inserted into the supply hole 44.

Therefore, water supplied at a water level higher than the supply hole 44 may be guided into the circulation duct 320 along the backflow allowing portion 333.

The backflow allowing portion 333 may be disposed downward of the discharge pipe 334. As a result, water supplied from the discharge pipe 334 may be completely introduced into the drainage tank 40, and water excessively supplied above a water level corresponding to the supply hole 44 may be recovered into the circulation duct 320 along the backflow allowing portion 333.

As a result, the residual water treater 330 may continuously circulate condensate into the circulation duct 320 and the drainage tank 40.

Accordingly, even when the drainage tank 40 is full, water collected in the reservoir 326 may be continuously introduced into the drainage tank 40 without leaking to the outside.

That is, even when the drainage tank 40 is full, condensate collected in the drainage tank 40 may be blocked from leaking to the outside of the machine room 300.

In addition, even when the drainage tank 40 is full, separate from notifying the water level state of the drainage tank 40 to the outside, the laundry treating apparatus may operate normally. In addition, even when the drainage tank 40 becomes full during the operation of the laundry treating apparatus, management of the laundry may be completed without stopping the laundry treating apparatus.

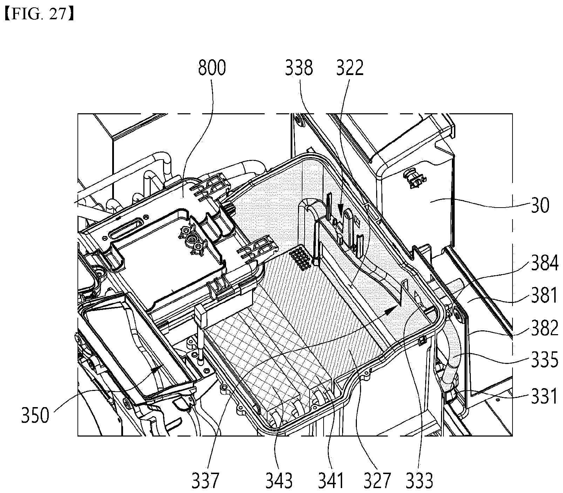

FIG. 27 shows a structure of a backflow allowing portion of a laundry treating apparatus of the present disclosure in detail.

The circulation duct 320 may have the evaporator 341 and the condenser 343 disposed therein. In this regard, the evaporator 341 may be disposed forward of the condenser 343 in the circulation duct 320.

The backflow allowing portion 333 may be disposed on a front surface of the circulation duct 320. In this regard, when water introduced into the circulation duct 320 from the backflow allowing portion 333 comes into contact with the evaporator 341 or the like, the evaporator 341 may be unnecessarily corroded or contaminated, and an efficiency of the evaporator 341 in cooling air may decrease.

Therefore, the evaporator 341 may be disposed closer to a rear side of the circulation duct 320 than the backflow allowing portion 333 such that water supplied from the backflow allowing portion 333 does not come into contact with the evaporator 341 along air directed toward the evaporator 341.

As a result, the water cover 327 may be exposed as it is between the evaporator 341 and the backflow allowing portion 333.

In addition, the evaporator 341 may be disposed to be spaced apart from the backflow allowing portion 333 by a width in the front and rear direction of the water cover 327.

The backflow allowing portion 333 or the discharge pipe 334 may be exposed forward of the circulation duct 320 by extending through the communicating portion 384. When the drainage tank 40 is seated on the detachment support 382, a distal end of the backflow allowing portion 333 or the discharge pipe 334 may be located inside the drainage tank 40.

The drain pipe 335 may be disposed between the detachment support 382 and the front surface of the circulation duct 320, and the drain pipe 335 may be disposed to connect the drainage pump 331 and the inlet pipe 332 to each other.

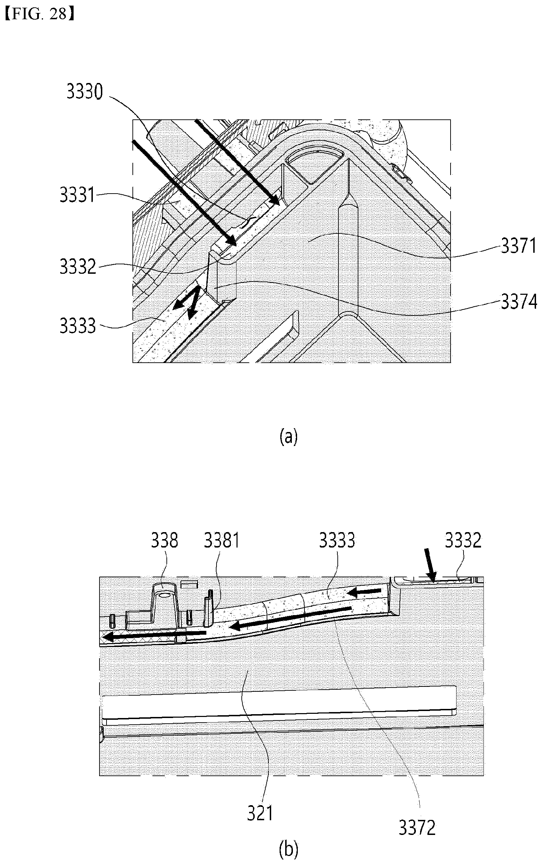

FIG. 28 shows a backflow allowing portion and a front surface of a circulation duct of a laundry treating apparatus of the present disclosure in detail.

Referring to (a) in FIG. 28, the backflow allowing portion 333 may include a backflow hole 3330 extending through the front surface of the circulation duct 320.

The backflow hole 3330 may be defined in an area that may at least partially face the supply hole 44. The backflow hole 3330 may be defined adjacent to one side surface of the circulation duct 320.

The backflow allowing portion 333 may further include a recovery flow channel 3331 that extends from the backflow hole 3330 defined in the front surface of the circulation duct 320 to be inserted into the supply hole 44 of the drainage tank 40.

The recovery flow channel 3331 may be formed in a duct shape having an open top surface and protruding both side surfaces in a longitudinal direction (the front and rear direction). Accordingly, water inside the drainage tank 40 may be introduced into the drainage tank 40 along the recovery flow channel 3331 without leaking to the outside.

The backflow allowing portion 333 may further include an inflow flow channel 3332 that protrudes inward from inside of the front surface of the circulation duct 320 to form a flow channel through which water introduced along the recovery path 3331 flows.

The inflow flow channel 3332 may block water introduced into the recovery flow channel 3331 from flowing downward along an inner surface of the circulation duct 320. Accordingly, recovered condensate may be prevented from being discharged to the outside air intake portion 322 or remaining in the front surface of the circulation duct 320.

The front surface of the circulation duct 320 may be formed such that an area where the backflow allowing portion 333 is disposed has a greater thickness than other areas because of the inflow path 3332, a flow channel to be described below, and the like. The circulation duct 320 may define a through-hole between the recovery flow channel 3331 and the inflow flow channel 3332 to receive water inside the drainage tank 40.

In one example, the drainage tank 40 may be disposed closer to one side surface of the circulation duct 320 than to the other side surface, and the supply hole 44 may be defined in the drainage tank 40 closer to one side surface of the circulation duct 320.

The recovery flow channel 3331 and the inflow flow channel 3332 may also be disposed closer to one side surface of the circulation duct 320. Accordingly, the residual water treater 330 may secure more flow channel space that may allow water introduced into the inflow flow channel 3332 to flow in the width direction of the circulation duct 320. Accordingly, water introduced into the inflow flow channel 3332 may secure a sufficient flow rate to flow along the width direction in the front surface of the circulation duct 320. Therefore, water may not remain in the residual water treater 330 and may be entirely collected back into the reservoir 326 along the residual water treater 330.

The inflow flow channel 3332 may have a width equal to or greater than that of the recovery flow channel 3331 such that water introduced into the recovery flow channel 3331 may flow along the width direction of the front surface of the circulation duct 320, and may have one end blocked at one side surface of the circulation duct 320 or blocked by a separate rib and the other end open.

The residual water treater 330 may further include a flow rate adjusting portion 3374 that may collect a certain amount of water in the inflow flow channel 3332 such that water may flow to the bottom surface of the circulation duct 320 after a sufficient flow rate is secured in the inflow flow channel 3332.

The flow rate adjusting portion 3374 may reduce a passage diameter of the inflow flow channel 3332.

The flow rate adjusting portion 3374 may be disposed in the width direction or a diameter direction, not a flow channel direction of the inflow flow channel 3332. The flow rate adjusting portion 3374 may be formed in a plate shape that extends from one surface of the inflow flow channel 3332 toward the other surface. For example, the flow rate adjusting portion 3374 may be disposed to face forward on rear one side surface of the inflow flow channel 3332.

The flow rate adjusting portion 3374 partially restricts the flow of water introduced into the inflow flow channel 3332, and thus is able to be viewed as a component of a blocking portion 337 to be described later.

The blocking portion 337 may block water introduced into the inflow flow channel 3332 from flowing to the evaporator 371.

The blocking portion 337 may be equipped as a partition wall or a step that extends higher than a flow channel on one surface of the flow channel including the inflow flow channel 3332 facing the evaporator 371.

The blocking portion 337 may be formed integrally with the circulation duct 320.

The blocking portion 337 may be disposed on an outer surface of the flow channel forming the backflow allowing portion 333 to prevent water flowing through the backflow allowing portion 333 from deviating from the backflow allowing portion 333.

For example, the blocking portion 337 may include a recovery partition wall 3371 disposed on a distal end of the inflow flow channel 3332 or on one surface of the inflow flow channel 3332 facing the evaporator 371.

The recovery partition wall 3371 may be formed in a shape of a partition wall that is spaced apart from the through-hole of the circulation duct 320, in which the recovery flow channel 3331 or the inflow flow channel 3332 is disposed, toward the evaporator 371 and shields the through-hole.

The recovery partition wall 3371 may be disposed to be spaced apart from the front surface of the circulation duct 320 by a width of the inflow flow channel 3332, and may face the supply hole 44.

The recovery partition wall 3371 may have an area size greater than that of the through-hole of the circulation duct. The recovery partition wall 3371 may extend from an outer surface or a rear surface of the inflow flow channel 3332 to one side surface of the circulation duct 320.

Water introduced into the inflow flow channel 3332 may be blocked by the recovery partition wall 3371 and flow along an extension direction of the inflow flow channel 3332. Therefore, water introduced into the inflow flow channel 3332 may be blocked from flowing to the evaporator 371.

Water introduced into the inflow flow channel 3332 may flow by passing through the flow rate adjusting portion 3374 after being collected in a certain amount by the recovery partition wall 3371 and the flow rate adjusting portion 3374.

Referring to (b) in FIG. 28, the backflow allowing portion 333 may include a guide flow channel 3333 that extends along the width direction of the circulation duct 320 from the inflow flow channel 3332. The guide flow channel 3333 may be disposed in an inner surface of the front surface of the circulation duct 320, and may have a thickness corresponding to that of the inflow flow channel 3332.

The guide flow channel 3333 may include an area that is defined to be lowered as it gets farther away from the inflow flow channel 3332. In addition, an inlet of the guide flow channel 3333, where the guide flow channel 3333 and the inflow flow channel 3332 are connected to each other, may be disposed higher than an outlet of the guide flow channel 3333.

As a result, water introduced into the guide flow channel 3333 may flow along the width direction of the circulation duct 320 by gravity.

In one example, a sensor installation portion 338 where the water level sensor that senses the water level of the water supply tank 30, a power device that opens and closes the outside air duct 322, and the like may be fixed may be further disposed inside the circulation duct 320. The sensor installation portion 338 may extend from an exposed surface of the guide flow channel 333.

The guide flow channel 3333 may have a width greater than that of the sensor installation portion 338.

The sensor installation portion 338 may further include a sensor partition wall 3381 disposed upstream of the sensor installation portion 338 or spaced apart therefrom toward the inflow flow channel 3332 to block water introduced from the guide flow channel 3333 from being introduced into the sensor installation portion 338 at a high flow rate. The sensor partition wall 3381 may have a width smaller than that of the guide flow channel 3333.

The guide flow channel 3333 may guide water introduced from the inflow flow channel 3332 directly to an upper portion of the reservoir 326 of the circulation duct 320. As a result, an amount of water introduced from the inflow flow channel 3332 remaining inside the circulation duct 320 may be minimized.

The guide flow channel 3333 may have a variable curvature or inclination along an extension direction thereof. The guide flow channel 3333 may have a steeper inclination in an area adjacent to the inflow flow channel 3332 than in an area furthest from the inflow flow channel 3332. As a result, water introduced into the guide flow channel 3333 may be prevented from remaining in the guide flow channel 3333.

The blocking portion 337 may include a guide partition wall 3372 that is disposed along an inner surface or one surface of the guide flow channel 3333 facing the evaporator 371 to have a height greater than that of the inside of the guide flow channel 3333.

The guide partition wall 3372 may prevent water flowing in the guide flow channel 3333 from deviating to the outside of the guide flow channel 3333.

The guide partition wall 3372 may correspond to the highest portion of the guide flow channel 3333, may extend along a longitudinal direction of the guide flow channel 3333, and may form the outer surface of the guide flow channel 3333.

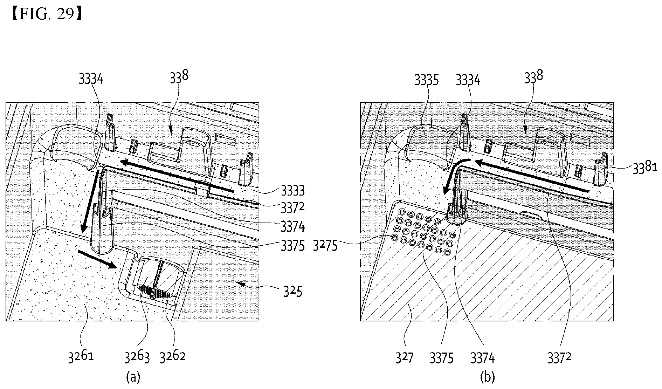

FIG. 29 shows a downstream of the backflow allowing portion.

(a) in FIG. 29 shows a structure of guiding water to the bottom surface of the circulation duct 320.

The guide flow channel 3333 may extend from the inflow flow channel 3332 to the reservoir 326.

The backflow allowing portion 333 may include a discharge flow channel 3334 extending from a distal end of the guide flow channel 3333 and guiding water flowing along the guide flow channel 3333 to the reservoir 326.

The discharge flow channel 3334 may guide water flowing through the guide flow channel 3333 toward the bottom surface of the reservoir 326. The discharge flow channel 3334 may extend downward along the inner surface of the front surface of the circulation duct 320 from the guide flow channel 3333.

In addition, the discharge flow channel 3334 may be formed as a length of a distal end of the guide partition wall 3372 formed in the guide flow channel 3333 is smaller than a length of the guide flow channel 3333.

The discharge flow channel 3334 may be formed as a width of the guide partition wall 3372 is smaller than a width of the front surface of the circulation duct 320 at the inner step surface of the front surface of the circulation duct 320 where the guide flow channel 3333 is disposed.

That is, the discharge flow channel 3334 may be formed between the guide partition wall 3372 and the other side surface of the circulation duct 320.

Water discharged from the discharge flow channel 3334 may flow downward along the inner surface of the front surface of the circulation duct 320 and may be collected in the reservoir 326. Condensate collected in the reservoir 326 may flow along the reservoir bottom surface 3261 to the recessed portion 3262. Condensate may flow again along the drain pipe 3263 to the drainage pump 331.

The blocking portion 337 may further include a discharge partition wall 3373 that guides water flowing along the guide flow channel 3333 to the reservoir 327.

The discharge partition wall 3373 may protrude from the inner surface of the circulation duct 320 to extend from a distal end of the guide partition wall 3372 toward the reservoir bottom surface 3261. The discharge partition wall 3373 may extend in a height direction from the inner surface of the circulation duct 320 and may extend up to the reservoir bottom surface 261.

In one example, the backflow allowing portion 333 may further include a flow channel restricting portion 3335 that protrudes from the guide flow channel 3333.

The flow channel restricting portion 3335 may be disposed at a certain distance from the distal end of the guide partition wall 3372 toward the other side surface of the circulation duct 320.

The guide flow channel 3333 and the inflow flow channel 3332 may be formed to protrude while forming a step on the inner surface of the circulation duct 320, and the flow channel restricting portion 3335 may be formed to protrude upward from the step surface (a top surface).

The discharge flow channel 3334 may be disposed between the flow channel restricting portion 3335 and the guide flow channel 3333, and may be formed between the flow channel restricting portion 3335 and the guide partition wall 3332.

The flow channel restricting portion 3335 may have the same width as the guide flow channel 3333.

The flow channel restricting portion 3335 may be disposed at a certain distance from the other side surface of the circulation duct 320 toward the inflow flow channel 3332. As a result, water flowing through the guide flow channel 3333 may quickly flow to the reservoir 326.

(b) in FIG. 29 shows that the water cover 327 is seated on the bottom surface of the circulation duct 320.

The water cover 372 shields the reservoir 326 and maintains the bottom surface of the circulation duct 320 flat. As a result, the backflow allowing portion 333 may allow water flowing along the guide flow channel 3333 to the water cover 327.

The water cover 372 may include a water outlet 3275 that allows condensate to pass therethrough in an area facing the discharge flow channel 3334. Water dropped onto the water cover 327 may flow to the reservoir 326 via the water outlet 3275.

In one example, the water cover 327 may be seated on the bottom surface of the circulation duct 320, but may have a width smaller than the width of the circulation duct 320 in consideration of assembly tolerance or the like so as to be easily withdrawn or installed in the circulation duct 320. However, because of the tolerance, the water cover 327 may shake and generate noise or the like when the laundry treating apparatus operates, or may be installed crookedly.

To prevent such problem, the discharge partition wall 3374 may further include a cover fixing stand 3375 with an expanded thickness. The cover fixing stand 3375 may have a thickness greater than that of the discharge partition wall 3374.

The water cover 327 may have a groove in a front edge into which the cover fixing stand 3374 may be inserted. As a result, the cover fixing stand 3374 may be seated in the groove of the water cover 327. As a result, the water cover 327 may be prevented from shaking or vibrating inside the circulation duct 320.

The cover fixing stand 3375 may be disposed to be spaced downwardly apart from an upper end of the discharge partition wall 3374. As a result, the groove of the water cover 327 may be easily secured to the cover fixing stand 3375.

In addition, the cover fixing stand 3375 may extend up to the bottom surface of the reservoir 326, and may have a thickness that increases in an upward direction.

An exposed surface of the cover fixing stand 3375 may be formed in a curved shape. Accordingly, the water cover 327 may be easily installed in or separated from the circulation duct 320 without being restricted by the cover fixing stand 3375.

The present disclosure may be modified and implemented in various forms, so that the scope of rights thereof is not limited to the above-described embodiments. Therefore, when the modified embodiment includes elements of the claims of the present disclosure, it should be considered to fall within the scope of the present disclosure.

## Claims

1. A laundry treating apparatus comprising:
a cabinet having an opening defined at a front side thereof;
an inner casing disposed inside the cabinet to accommodate laundry therein;
a door pivotably coupled to the cabinet and configured to open and close the opening;
a circulation duct circulating air discharged from the inner casing;
a heat exchanger disposed inside the circulation duct and configured to cool and heat air;
a drainage tank disposed forward of the circulation duct and storing water condensed from the heat exchanger; and
a residual water treater configured to collect water condensed from the circulation duct into the drainage tank,
wherein the residual water treater allows the drainage tank and inside of the circulation duct to be in communication with each other such that a portion of water collected in the drainage tank is dischargeable back into the circulation duct.

2. The laundry treating apparatus of claim 1, wherein the residual water treater includes a backflow allowing portion extending through one surface of the circulation duct and in communication with the drainage tank.

3. The laundry treating apparatus of claim 2, wherein the backflow allowing portion is disposed at a certain height apart from a bottom surface of the drainage tank.

4. The laundry treating apparatus of claim 3, wherein the residual water treater includes:
a drainage pump configured to allow water collected on a bottom surface of the circulation duct to flow; and
a discharge pipe in communication with the drainage pump and the drainage tank to inject water into the drainage tank, wherein the backflow allowing portion is equipped separately from the discharge pipe.

5. The laundry treating apparatus of claim 4, wherein the backflow allowing portion and the discharge pipe are blocked from being in direct communication with each other.

6. The laundry treating apparatus of claim 2, wherein the backflow allowing portion further includes:
a backflow hole defined to extend through the circulation duct; and
a recovery flow channel extending from the backflow hole to be inserted into the drainage tank to allow the backflow allowing portion and the drainage tank to be in communication with each other.

7. The laundry treating apparatus of claim 6, wherein the backflow allowing portion includes a guide flow channel extending in a width direction of an inner surface of the circulation duct from the backflow hole to guide water to a bottom surface of the circulation duct.

8. The laundry treating apparatus of claim 7, wherein the circulation duct includes a reservoir recessed from the bottom surface thereof to collect water therein,
wherein the drainage tank or the backflow hole is disposed close to one side of the inner surface of the circulation duct,
wherein the reservoir is disposed close to an opposite side to the one side of the inner surface of the circulation duct,
wherein the guide flow channel extends from the backflow hole toward the reservoir.

9. The laundry treating apparatus of claim 7, wherein the guide flow channel extends to be lowered along the width direction of the inner surface of the circulation duct.

10. The laundry treating apparatus of claim 9, wherein the backflow allowing portion further includes:
an inflow flow channel extending from the backflow hole to one end of the guide flow channel; and
a discharge flow channel extending from the other end of the guide flow channel to the bottom surface of the circulation duct.

11. The laundry treating apparatus of claim 10, wherein the discharge flow channel is set to have a steeper inclination than the guide flow channel,
wherein the backflow allowing portion further includes a flow channel restricting portion protruding from a bottom surface of the guide flow channel and inducing water flowing along the guide flow channel to flow toward the discharge flow channel.

12. The laundry treating apparatus of claim 1, wherein the residual water treater is disposed on a front surface of the circulation duct facing the heat exchanger.

13. The laundry treating apparatus of claim 12, wherein the residual water treater includes:
a backflow allowing portion extending through the front surface of the circulation duct and receiving water from the drainage tank; and
a blocking portion blocking water flowing along the backflow allowing portion from flowing to the heat exchanger.

14. The laundry treating apparatus of claim 13, wherein the backflow allowing portion guides water introduced from the drainage tank to a bottom surface of the circulation duct at the front surface of the circulation duct,
wherein the blocking portion is disposed along an edge of the backflow allowing portion exposed to an inner surface of the circulation duct to block water from being introduced into the heat exchanger.

15. The laundry treating apparatus of claim 13, wherein the backflow allowing portion includes:
a backflow hole extending through the front surface of the circulation duct and in communication with the drainage tank; and
an inflow flow channel extending from the backflow hole to the inner surface of the circulation duct,
wherein the blocking portion includes:
a recovery partition wall extending from a distal end of the inflow flow channel to face the evaporator; and
a flow rate adjusting portion extending from the recovery partition wall toward the front surface of the circulation duct to collect a portion of water introduced from the backflow hole.

16. The laundry treating apparatus of claim 2, wherein the backflow allowing portion is formed integrally with the circulation duct.
